(19) Europäisches Patentamt
European Patent Office
Office européen des brevets

(11) EP 3 215 837 B1

(12) **EUROPEAN PATENT SPECIFICATION**

(45) Date of publication and mention of the grant of the patent:
**03.01.2024 Bulletin 2024/01**

(21) Application number: **15794247.5**

(22) Date of filing: **05.11.2015**

(51) International Patent Classification (IPC):
***G01N 27/327*** *(2006.01)*

(52) Cooperative Patent Classification (CPC):
**G01N 27/3274; G01N 27/3272**

(86) International application number:
**PCT/GB2015/053356**

(87) International publication number:
**WO 2016/071698 (12.05.2016 Gazette 2016/19)**

(54) **METHOD OF USING A TEST DEVICE**

VERFAHREN ZUR VERWENDUNG EINER TESTVORRICHTUNG

PROCÉDÉ D'UTILISATION D'UN DISPOSITIF DE TEST

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.11.2014 GB 201419799**

(43) Date of publication of application:
**13.09.2017 Bulletin 2017/37**

(73) Proprietor: **Inside Biometrics International Limited**
**Dingwall, Scotland IV15 9QF (GB)**

(72) Inventors:
- **RODGERS, James Iain**
**Dingwall IV15 9QF (GB)**
- **LEACH, Christopher Philip**
**Dingwall IV15 9QF (GB)**
- **CARDOSI, Marco Fabio**
**Dingwall IV15 9QF (GB)**

(74) Representative: **Marks & Clerk LLP**
**15 Fetter Lane**
**London EC4A 1BW (GB)**

(56) References cited:

| | |
|---|---|
| EP-A2- 0 537 761 | EP-A2- 2 333 542 |
| EP-A2- 2 444 500 | WO-A1-2010/085271 |
| WO-A1-2016/067037 | US-A1- 2004 180 391 |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Field of the Invention

The present invention relates to a method of using a test device. The test device is an electrochemical test strip as used with bodily fluid monitors for determining the concentration of analytes in an individual's bodily fluid sample.

### Background to the Invention

In the field of diagnostic devices such as used in the medical device industry, especially those used for analysing blood or other bodily fluid samples, it is often required for users to monitor biometrics such as the levels of certain chemicals, substances, or analytes present for example in their bloodstream. For instance, diabetics in particular must regularly monitor the concentrations of glucose in their blood in order to determine if they are in need of insulin. In order to respond effectively to an individual's needs to monitor blood sugar levels, diagnostic devices and kits have been developed over the years to allow an individual to autonomously determine the concentration of glucose in their bloodstream, in order to better anticipate the onset of hyperglycaemia or hypoglycaemia and take preventative action as necessary. The existence of such diagnostic devices places less strain on the healthcare system at large, as patients are able to administer insulin in their own home and without having to do so in the presence of a medical professional.

Typically, the patient will perform a finger stick to extract a small drop of blood from a finger or alternative site, using a lancing device. An electrochemical test device, which is often a strip, is then inserted into a diagnostic meter, and the sample is applied on the test strip. Through capillary action, the sample flows across a measurement chamber of the device and into contact with one or more electrodes or similar conductive elements coated with sensing chemistry for interacting with a particular analyte or other specific chemical (for example glucose) in the blood sample. The magnitude of the reaction is dependent on the concentration of the analyte in the blood sample. The diagnostic meter may detect the current generated by the reaction of the reagent with the analyte, and the result can be displayed to the user.

Typically, such electrochemical test devices have a working electrode and a counter/reference electrode, and may have more than one working electrode. EP2056107 discloses a test strip which has two working electrodes which measure the same analyte. This allows the analyte to be measured twice so that the two measurements can be compared so that each can be used as a check for the other. This allows detection of inadequate fill for the strip or of a manufacturing defect if the two measurements are significantly different. US 2007/0131549 discloses a test strip which has two working electrodes, one of which measures glucose using glucose oxidase and the other of which measures glucose using glucose dehydrogenase. The two electrodes respond to glucose over the entire linear range. If the sample has a lower partial pressure of oxygen, the glucose oxidase-based electrode will give a higher response, while the glucose oxidase-based electrode will give an accurate result. In these circumstances the reading from the glucose oxidase-based electrode is preferred. US2004/180391A1 proposes in vivo or in vitro monitoring of chemical and biochemical species. EP0537761A2 proposes a biosensor. EP2444500A2 proposes a method for measuring a target object in a sample by using an oxidase.

Against this background there remains a need for improved methods of using test devices such that more accurate measurements may be derived therefrom. In particular there remains a need to more accurately measure a current generated by a test device when the test device reacts with a fluid sample, so as to more accurately determine an amount of analyte within the sample.

### Summary of the Invention

The invention is defined in the accompanying claims. There is provided a method of using a test device for measuring the amount of analyte in a fluid sample comprising a substrate. The substrate has thereon a first analyte reagent formulated to react with the analyte to generate a signal indicative of the presence or amount of analyte in the sample. The first analyte reagent has a first time-based response characteristic. The substrate also has thereon a second analyte reagent formulated to react with the analyte to generate a signal indicative of the presence or amount of analyte in the sample. The second analyte reagent has a second time-based response characteristic.

The first and second analyte reagents solvate differently when brought into contact with the sample, thereby producing analyte-dependent signals with different current vs time characteristics. Relatively fast solvating reagents are more precise with respect to eliminating effects of manufacturing variation but more susceptible to systematic sample errors. Where the sample to be tested is blood, a systematic sample error may be haematocrit. On the other hand, relatively slower solvating reagents are less precise with respect to effects of manufacturing variations but less susceptible to systematic sample errors. For example, a fast solvating reagent would equilibrate within about 10% of the total test time (e.g. the period during which currents at the electrodes are measured), whereas slower solvating reagents would not have fully equilibrated within about >20% of the total test time. The total test time may be of the order of 5-15s typically, but other test times may be used. The time based response characteristic for each electrode is established by taking current readings at one or more time points within the course of the measurement, and preferably taking readings from multiple time points from within the test, as the reagent pad progresses through different physical stages, while the underlying signal generating chemical reactions are proceeding.

For the purposes of this invention, one or more time based readings may be taken simply as is to constitute the time based response characteristic. Because the underlying signal generating chemical and electrochemical reactions and processes at both electrodes are well understood, and may be ostensibly similar, whereas the different physical stages through which the sensor pads progress are subject to complex interactions with the sample, it has been discovered that, with appropriate formulations, the observed relative differences between the time based response signal characteristic of the two analyte reagents can be used to elucidate and correct for systematic errors, while maximising precision, by means of suitable algorithms. The respective analyte reagent compositions are designed to have different diffusional, solvation and/or dissolutional behaviour characteristics in order that they generate a signal with different time-based response characteristics.

Typically, a reagent pad will initially undergo a 'solvation' stage during which the dominant process will involve solvent (e.g. water, or plasma) from the sample solution penetrating into the reagent pad, with the underlying chemical reactions being largely confined therein. The reagent pad may subsequently swell and a dissolution stage may be reached, wherein the dominant process becomes solute materials from the reagent pad spreading out into the bulk sample layer (dissolution defined herein as the process of pad solute component penetrating out into the bulk sample). Reactions will occur in both pad and bulk domains whilst dissolution is progressing. And ultimately an equilibration stage may be reached wherein largely complete dissolution has been effected and the pad and bulk domains have become stable and may be essentially uniform. The underlying chemical reactions will continue, but will be largely unaffected by physical interchange of components between a reagent pad and a bulk sample layer, which will have reached a stable equilibrium, with the response signal subsequently being controlled solely by the underlying diffusion limited reaction kinetics of the reactive species.

For clarity of meaning, the 3 stages are described above as discrete sequential steps, however in reality they will be overlapping to some extent with transitional stages occurring between them which have elements and characteristic of each. Moreover, the different subcomponents of the reagent pads may progress through the stages at different rates, such that the behaviour of the reagent pad as a whole becomes a product of the behaviour of its individual sub components. Thus, altering the formulation of the sub components within the reagent pad becomes identifiable to one skilled in the art as a suitable means by which the time based progress through these stages of any given reagent pad may be designed and controlled.

Furthermore, for a given individual reagent pad formulation and composition within the specific duration of a given test, one of the stages may predominate throughput the course of the test to an extent such that the other stages are to all intents and purposes absent. Alternatively, for any individual pad formulation and composition within the specific duration of a given test, one of the stages may be minimal to the extent that it alone is to all intents and purposes absent.

In the case of a fast solvating system, the equilibration stage described above may be beginning to predominate in as little as ~10-25% of the total test time, and for example 0.5-2.5s or thereabouts, and the solvation stage may be effectively absent to all intents and purposes. In this instance the most useful part of the time based response characteristic will be the portion beyond about 2-3s, preferably beyond about 3-5s and most preferably beyond about 5s, as this will enable the measurement result to be derived with optimum precision since the response signal will be controlled by the underlying diffusion limited reaction kinetics of the reactive species in this region, and inconsistencies generated during the sensor manufacture will be maximally equilibrated away.

Within the algorithms employed, there may still be some useful information contained prior to 2-3s, preferably prior to 3-4s and most preferably prior to 5s, because even though limited and noisy sample correction information exists at these time points in such formulations, when combined algorithmically with the stronger correction signals from slower solvating systems beneficial correction and cross validations may still result. However time based response characteristic from this region would generally be downweighted relative to the time based response characteristic from beyond about 2-3s, more preferably beyond about 3-5s and most preferable beyond about 5s, when deriving a final measurement.

In the case of a slower solvating system, the solvation and dissolution stages described above may predominate for at least about 20% of the total test time, and for example >1.5-3s or thereabouts, and the full equilibration stage may not be reached until at least about 40% of the total test time, for example 3s-5s or thereabouts more preferably after 5s or even beyond the duration of the total test time. In this instance the first most useful parts of the time based response characteristic will be the portion beyond about 0.5-1s, preferably between about 1s and 5s as this will enable the measurement result to be derived with the optimum trade-off between precision and sample error (for example) haematocrit related information, since the response signal will be effected by the changing interaction between pad components and the sample components, and time based response changes generated as a result of minimal exposure to interfering sample components (during solvation), and maximum differential changes resulting from progressively increasing exposure to interfering sample components (during dissolution) will be most abundant in this portion of the time based response characteristic.

The second most useful part of the time based response characteristic from a slow solvating system will be the portion closest to the end of the test, preferably beyond around 5s, more preferably around 7s to 10s. At this point the dissolution will be nearest to the point of equilibration that can be reached within the test for that electrode composition, and it will be at its most precise for that formulation, but also its relative similarity to the behaviour observed from an adjacent fast solvating electrode system exposed to the same analyte will be maximised. Algorithms can thereby be created in which the measurement signal is maximised through combination, while the error signals are minimised through differential comparison using the weighted combination of suitable time based response characteristics simultaneously generated from the same sample across multiple electrodes within the same test strip, which have been deliberately designed and/or formulated to produce different time based response characteristics from the same sample.

Other response characteristics that could influence the time-based response include reagent pad height, reagent pad micro-structure, transit time of sample into testing chamber, pad porosity, hydrodynamic properties of the fluid during the measurement, co-solvency effects, pH effects and buffering level, surface energy of the reagent pad, outgassing of the reagent pad, relative areas of the reagent pads, thickness variation between the reagent pads, enzymatic inhibitors within the enzyme pad, electrochemical additives within the reagent pad, and electrochemical catalysts and/or inhibitors within the reagent pad.

The test device is an electrochemical test measurement device comprising two working electrodes, one comprising the first analyte reagent and the other comprising the second analyte reagent. The respective reagents may be formed in layers on the electrode which are designed and formulated to have significantly different diffusional, solvation and/or dissolutional response characteristics.

In addition, the test device may comprise a counter electrode.

A first working electrode may comprise a first analyte reagent that has relatively rapid dissolution, solvation and/or diffusional exchange and equilibration with the sample. Such a reagent will rapidly produce a reading which is largely independent of defects and deviations which arise during manufacture of the test strip, and which therefore demonstrates reduced inconsistency of results between different test strips. This electrode is effective at minimising precision errors within and between electrochemical test measurement strips. However, this electrode's response is thereby inevitably prone to suffer from inaccuracies arising from systematic differences and variations within the solvation, diffusional and solvency properties of the sample containing the analyte. A non-limiting example of such a systematic sample difference that would produce such inaccuracies includes variation within and between individual test subjects in the packed red blood cell volume (haematocrit), in situations where the sample is blood.

A second working electrode may comprise a second analyte reagent that has a more progressive equilibration with the sample than the first reagent analyte. The components within this reagent will generally equilibrate with the bulk sample more gradually. As a result, this reagent is more susceptible to manufacturing variations and the inaccuracies attendant therein. However, it maintains a greater degree of diffusional limitation within the reagent, with penetration of the bulk sample components into the reagent and dispersion of reagent components into the bulk being more gradual. Consequently, this electrode is - initially at least - much less susceptible to the systematic difference arising as a result of the diffusional properties of the bulk sample, e.g. those differences arising from variations in the packed red blood cell volume in situations where the sample is blood.

The first and second analyte reagents comprise an enzyme and a mediator. The analyte is glucose, the enzyme may be glucose oxidase or a glucose dehydrogenase and the mediator may be potassium hexacyanoferrate (III). Other suitable enzymes and mediators for the detection of glucose and other analytes are known to those of skill in the art. For example, the mediator may be selected from hexaamine Ru (III) chloride salts and derivatives thereof, ferrocene/ferrocinium mediators (including water soluble polymeric ferrocence mediators and water soluble ferrocene salts), phenothiazine mediators, and quinone mediators, such as tetrathiafulvalene, diimines, thionine oxometalates, polymetallophthalocyanines, pyrroloquinoline quinone, fluorenones, and quinonoid redox dyes such as indamines, phenazines, and phenoxazines.

In addition to these components, the reagents comprise film-forming components and non-film-forming components. By adjusting the relative proportion of these components, the respective analyte reagents are adjusted so that they provide the signal indicative of the presence or amount of analyte within a chosen time period. A high proportion of non-film-forming components relative to film-forming components provides a reagent that dissolves rapidly and causes the creation of a signal after a relatively short time period. Conversely, a high proportion of film-forming components relative to non-film-forming components provides a reagent that dissolves more slowly and causes the creation of a signal after a relatively long time period. The creation of reagents that dissolve within different time periods is within the ambit of the skilled person. Film forming components with a high density (or specific gravity) will need to be present at a greater level than equivalent components with a lower density (or specific gravity) to achieve the same effect on solvation properties, all else being equal. Similarly non Film forming components with a high density or specific gravity will need to be present at a greater level than equivalent non film forming components with a lower density (or specific gravity) to achieve the same effect on solvation properties, all else being equal.

Additionally, film forming components with a natural propensity to dissolve slowly can be incorporated at a lower level than film forming components with a natural propensity to dissolve more quickly to achieve the same effect on solvation properties all else being equal. Whereas non film forming components with a natural propensity to dissolve more slowly or not at all need to be incorporated at a higher level than non-film formers with a natural propensity to dissolve quickly to achieve the same effect on solvation properties all else being equal.

Notwithstanding the above, where both film formers and non-film formers dissolve rapidly and where the specific gravity of the film forming components is about 1 and the specific gravity of the non-film forming components is about 1.8, a fast dissolving reagent formulation would, when in the dry state, generally comprise a ratio of film forming components to non-film forming components of less than 1:1.1, more preferably less than 1:2 and most preferably about 1:3 or less. A slow dissolving reagent formulation would, when in the dry state, generally comprise a ratio of film forming to non-film forming components of 1:1 or greater, and more preferably around 3:2 or more.

Film-forming components include any material that, when cast from solution onto a surface and then dried, exhibits a tendency to form a layer which effectively minimises the surface area at the solid/air interface in order to correspondingly minimise the free surface energy thereat, producing a tendency towards flat and even film layers. An example of one such material is hydroxyethycellulose (HEC), but it will be apparent that other hydrophilic film forming polymers exist which would work as suitable replacements, such as polyvinyl alcohol (PVA), polyethylene oxide (PEO), pullulans, carboxymethylcellulose (CMC), acrylates, water soluble polyesters and polyols, dextrans, xantham gums and similar materials. Preferably, the film-forming components have the following properties: aqueous solubility, viscosity at the desired concentration (with regard to coating performance), and compatibility with other components of the analyte reagent. Higher molecular weight/viscosity grades of polymer can be utilised at lower levels to maintain similar wet reagent behaviour whilst reducing the overall amount of film-forming component present relative to other components. Conversely, lower viscosity/molecular weight analogues at higher levels can be utilised to maintain a suitable coating viscosity for the analyte reagent whilst increasing the ratio of film-forming component to other components. Thus strategies for modulating the levels of film-forming component relative to other components, while maintaining similar printability behaviour, become apparent to those skilled in the art, as means by which the solvation properties of the analyte reagent layer may be controlled to within desirable limits. Similarly, different polymers possessing greater or less solubility can be used as a means of affecting the solvation properties of the analyte reagent.

Non-film forming components include crystallising materials that disrupt film formation. The processes of crystal formation - for example - can thermodynamically predominate over the minimisation of surface energy at the resulting solid air interface, such that domains of crystal growth extend within and protrude from the film surface, increasing the surface area of the solid/air interface well beyond its minimum, and wherein compositional differences between such crystalline domains and adjacent domains (containing other components) can arise spontaneously through the thermodynamically driven association of like constituents. Examples of rapidly solvating crystallising material include inorganic salts such as potassium phosphate, sodium chloride, potassium chloride, nitrate salts, ammonium salts, organic materials such as sucrose, citrates, ethane-sulfonic-acid salts such as PIPES, HEPES, MES, or any other rapidly solvating crystalline material. The electrochemical mediator may also act as a non-film-forming component.

The first and second analyte reagents may comprise a surfactant. Typically these are employed to ease the application of the analyte reagent to the substrate. The choice of surfactant and the level at which it is employed can also influence the wettability of the film and the penetration of sample components thereunto. Using different surfactants and/or surfactant combinations to speed up or slow down penetration of the sample into the reagent will be recognised as a viable strategy by which the analyte reagent solvation properties might also be influenced. Those skilled in the art can select a suitable surfactant. Examples include Tergitol NP-9, Triton™ X100; Borchi Gol™LA200, 1375, LA6; Brij™35; Pluronic™P103; Tergitol™ NP-7.

The substrate of the device of the present invention may comprise a base substrate material layer which may be, for example, polymer (PET) or board or coated paper. The base substrate material layer may have a patterned conductive electrode layer deposited thereon, for example by means of screen printing conductive carbon-graphite layers, by metal layer deposition means using Au, Pd, Pt or other suitable electrode conductors known to those skilled in the art.

As mentioned, the analyte is glucose. The sample may be blood. A particular problem with blood is the effect haematocrit can have on measuring the level of analyte therein. The packed red blood cell volume can vary between samples affecting the ability of the analyte to come into contact with the analyte reagent. The presence of first and second analyte reagents aims to mitigate the effect of haematocrit on the detection and measurement of analyte. Alternatively, the sample may be urine, interstitial fluid, plasma, serum, saliva or spinal fluid. Still other fluid samples may be used.

We disclose herein a method of using the test device. The method comprises polarising the first and second working electrodes with respect to a counter/reference electrode. For example, a potential difference is applied between the first (working) electrode and the counter/reference electrode, as well as between the second (working) electrode and the counter/reference electrode. The method further comprises applying the fluid sample to the test device; measuring one or more first currents at the first working electrode; measuring one or more second currents at the second working electrode; and using the one or more first and second currents to determine a corrected current measurement.

The method further comprises determining an amount of analyte in the fluid sample based on the corrected current measurement. By using a set of currents generated at the first working electrode with a set of currents generated at the second working, a more representative overall current measurement may be determined, and one which may be used to more accurately determine an amount of analyte in the sample.

The one or more first currents are measured over a first time period. The one or more second currents are measured over a second time period and a third time period. In some embodiments the first time period may overlap with the second and/or the third time period. The one or more first and second currents may be measured over a test time period, which may comprise the first, second and third time periods in their entirety.

The first time period begins when the first analyte reagent is substantially completely dissociated or dissolved within the fluid sample.

The first time period may be chosen to be long enough that the precision on the first analyte reagent has been maximised, and the interval may be such that the average is less susceptible to random noise. Ideally, any time point after the analyte reagent has fully dissociated is appropriate. The faster the analyte reagent dissolves, dissociates and equilibrates, the sooner the first time period may begin. At the end of the first time period, the dissolution of the analyte reagent should be largely complete.

The second time period begins when the second analyte reagent begins to dissociate or dissolve within the fluid sample.

The second time period may be chosen so as to maximise the amount of haematocrit information that is available, to minimise the raw haematocrit effect whilst trying to avoid regions where the imprecision is so high as to confound the useful information obtained. Very early time points tend to be noisy and are generally affected by other system parameters (e.g. fill speed). Therefore, by using a slower dissolution chemistry, information from an 'early' stage of the dissolution process can be obtained with reasonable precision at later time points than would be possible with a relatively fast dissolution chemistry (as in the first analyte reagent).

The second time period may therefore begin when the second analyte reagent has solvated enough to give an analyte-sensitive response but where matrix interference is minimised because solvation is still at a relatively early stage. If the second time period begins too soon, the correction effect may get lost in noise as the measurement reactions have not built up sufficient momentum. If on the other hand the second time period begins too late, the system may already be equilibrated too far and the correction information may be confounded with the current measurements taken during the third time period, and haematocrit correction information may be lost.

If the second analyte reagent has a sufficiently slow dissolution rate, the second time period may commence after the first time period has begun, even though the start of the first time period represents a greater degree of dissolution, since the first time period relates to a relatively fast dissolving analyte reagent where dissolution generally occurs more rapidly.

The third time period begins when dissolution or dissociation of the second analyte reagent within the fluid sample is not complete.

The third time period generally begins when dissolution of the second analyte reagent is incomplete but the system is equilibrated, and a significant degree of haematocrit sensitivity is present. An aim is to select the second and third time periods to maximise the signal difference between the currents measured during the second period and the currents measured during the third period, whilst also (at the expense of precision) avoiding regions where the coefficient of variation is too high at relatively early time points.

The first, second and third time periods may be empirically optimized, but in general the optimum values are heavily influenced and controlled by the relative dissolution rates of the first and second analyte reagents. There is a trade-off between haematocrit and precision, versus the time taken for the assay. In other words, the desire to carry out the assay as quickly as possible should be balanced against the precision required.

In summary, the first time period begins when the first analyte reagent is completely dissolved and therefore the system is equilibrated. The second time period begins when the second analyte reagent begins to dissolve and the system is not yet equilibrated, though whilst adequate signal and precision are accessible. The third time period begins when dissolution of the second analyte reagent is equilibrated, but not necessarily complete. The duration of each time period may be chosen appropriately, for example to achieve desired averaging and smoothing.

Using the one or more first and second currents to determine the corrected current measurement comprises determining a first current average by averaging the one or more first currents; determining a second current average by taking a difference of a sum of the one or more second currents measured over the second time period and a sum of the one or more second currents measured over the third time period. This may be carried out for each of multiple test devices, thereby obtaining a set of second current averages.

By exposing the first and second analyte reagents to the same sample, it is possible to generate a system with improved measurement accuracy. The deliberately contrived difference in time-based response characteristic between the first and second analyte reagents is exploited using the algorithmic approach to generate a superior analyte measurement method.

## Brief Description of the Drawings

Specific embodiments of the invention will now be described in connection with the accompanying drawings, of which:

Figure 1 is a diagrammatic representation of a section through the working part of an electrochemical test strip used in a method of an embodiment of the present invention;
Figure 2 is a diagrammatic representation of a section through the working part of the electrochemical test strip before and after contact with the sample;
Figure 3 is a schematic model of the diffusional processes that happen externally and internally to the reagent pad during measurement and adjacent to the working electrode surface;
Figure 4 shows the cascade of events that occur during the different stages of the measurement;
Figure 5a shows the shape of the time-based characteristic response that would be observed from a relatively thin reagent pad composition that also underwent rapid solvation and near complete dissolution;
Figure 5b shows the shape changes of the time based response that would be observed in circumstances where the reagent layer was undergoing progressive solvation and incomplete dissolution and wherein the reagent pad thickness was being varied;
Figure 5c shows the impact of changing the diffusivity on the time-based response;
Figure 5d shows a plurality of the time-based characteristic responses that could result from the complex dynamics of a system under different analyte and sample conditions where the dissolution, solvation, diffusion and partitioning properties were changing over time within the measurement;
Figure 6a shows an example of the time-based characteristic responses observed when identical electrodes with the same reagent formulation are exposed to different concentrations of analyte, to produce a response whose magnitude can readily be used to effect a measurement of the analyte concentration by means of a suitable algorithm;
Figure 6b shows an example of the time-based responses observed when identical electrodes with the same reagent formulation are exposed to multiple samples with the same analyte concentration but where the sample haematocrit has been varied, to create a signal response whose measurement would ordinarily be prone to suffer from systematic inaccuracies because of the confounding effect of the changing haematocrit upon the time-based characteristic responses;
Figure 7 shows the process steps involved in producing a PCA (Principal Component Analysis) based measurement algorithm for the method of the present invention;
Figure 8 shows the coefficient matrix used to generate Principle Component scores from the results at test times used in an algorithm derived from and used with an embodiment of the present invention;
Figure 9 shows the coefficient matrix used to generate Principle Component scores from the results at test times used in an algorithm derived from and used with another embodiment of the present invention;
Figure 10a shows a three-dimensional plot of the calibrated measurement results produced according to an embodiment of the present invention, plotted against the haematocrit level and reference glucose concentration of the sample tested;
Figure 10b is a rotation of Figure 10a to show the glucose measurement performance according to an embodiment of the present invention;
Figure 10c is a rotation of Figure 10a, showing the insensitivity of the measurement across changes in haematocrit according to an embodiment of the present invention; and
Figure 10d shows the bias plots to haematocrit at different glucose levels of 660 individual test strips tested at different haematocrit and glucose combinations, and the percentage of these achieving an accuracy of within 15% of reference value at each of the test levels.

## Detailed Description of Specific Embodiments

Whilst various embodiments of the invention are described below, the invention is not limited to these embodiments, and variations of these embodiments may well fall within the scope of the invention which is to be limited only by the appended claims.

Referring to Figure 1, the working part of an electrochemical test used in the method of the present invention is built up from multiple functional layers and comprises a base substrate material layer 100 which may be, for example, polymer (PET) or board or coated paper. Onto this layer is deposited a patterned conductive electrode layer 200 for example by means of screen printing conductive carbon-graphite layers, by metal layer deposition means using Au, Pd.

Pt or other suitable electrode conductors known to those skilled in the art. A dielectric layer 300 over-coating the conductive layer 200 by which the electrochemically active area of the conductive layer 200 can be more precisely defined, may be included as shown. A reagent layer 400 is disposed above the conductive electrode layer 200, overlapping the optional dielectric layer 300.

Further reagent layers may be present. One or more reagents may be applied to each electrode of a test device. For example, a first reagent layer comprising a first reagent may be applied to a first working electrode of the test device and to a second working electrode of the test device. A second reagent layer comprising a second reagent may be applied to one of the first and second working electrodes of the test device, thereby ensuring that both the first and second reagents are provided to one of the working electrodes.

It will be recognised by one skilled in the art that each individual functional layer may optionally be built up of a plurality of individual layers and/or in a plurality of stages or process steps if so required, according to the need for optimally establishing required properties such as (i) the desired layer thickness, (ii) the electrical conductivity, (iii) the physical integrity, (iv) the uniformity of the layer, (v) the porosity, (vi) the surface area, (vii) the surface energy, or any desired gradation of such properties through the various functional layers.

The working electrode portion thus assembled is located within a sample void - for example - a sample capillary volume 500, and forms a component part of a complete working electrochemical measurement test strip. It is disposed such that the sample volume 500 immediately adjacent the reagent layer will be filled with sample liquid during use. This sample liquid in sample volume 500 will exchange components with the reagent layer 400, with components of the reagent layers dissolving and diffusing out into the bulk and elements of the sample fluid penetrating the reagent layer 400, and with components from both ultimately being able to react electrochemically at the surface of the conductive layer 200.

Figure 2 shows conceptually what happens to the reagent pad 400 as liquid sample is introduced to the sample void 500 at time $t_1$. Solvent and solutes from the sample penetrate the reagent pad. Components therein dissolve and diffuse. This has a swelling effect on the reagent pad. It also affects its diffusivity. As the reagent pad swells and components diffuse out into the bulk, inconsistencies created during the manufacturing process equilibrate out as this progresses to time $t_2$.

To understand the significance of such changes in the pad properties over time, it is useful to have a model of how the electrode response is actually derived and which reagent pad properties are critical in influencing this response. Just such a model is shown schematically in Figure 3.

The working electrode response is derived from a diffusion controlled process in which reduced (or, in an alternate case, oxidised) species derived from the reaction of analyte within the bulk sample diffuse towards the conductive layer 200 where they are electrochemically oxidised (or, in the alternate case, electrochemically reduced) and thereby removed from the system. It is this flux of species from the bulk towards the conductive layer 200 that ultimately controls the response signal which is measured at the working electrode.

Since the conductive layer 200 is effectively an infinitely reactive locus for such reduced (or, in the alternate case, oxidised) species, the concentration of reduced (or, in the alternate case, oxidised) species at this boundary is nominally maintained at zero. A concentration gradient (dc/dx) is thereby continually generated between this interface and the bulk layer with a net flux of oxidised species along this concentration gradient as per Fick's law. It will be apparent from Figure 1 that the system is actually comprised of 2 separate domains of flux, these being the bulk sample, and the reagent pad layer. Both of these domains will have different properties. The critical properties with respect to the diffusional flux that generates the electrode signal are:

1. Distance (x) between the domain and the surface of the electrode sink, which controls the concentration gradient (dc/dx) along which the diffusional flux is driven at any point in time.

2. Diffusivity (D) - the relative speed at which molecules can navigate the linear distances within that specific layer, which regulates the relative flux observed along any given concentration gradient.

3. Partitioning (p) - the relative extent to which a domain can act as a membrane and thereby exclude penetration by substances with any given physical property, for example, physical size, solvated volume/ Molecular weight, electrical charge/polarity.

The relative values that these properties have within the two separate domains (bulk & reagent pad) at different times throughout the measurement process will fundamentally influence the response signal which is derived therefrom.

It is typically expected that - with all else equal - the diffusivity within the pad would be lower than the diffusivity in the bulk since some pad components would act as an obstacle to be navigated by diffusing species, that the pad distance (x) is small and finite, whereas the bulk boundary is relatively large and may be semi-infinite and that the pad may selectively partition some species from within the sample, whereas the bulk layer will not partition species from within the sample, but it might act as a selective partition for some species diffusing out from the reagent pad.

This simple two domain model with the static parameters described already presents a complex analytical challenge with respect to predicting the actual time-based characteristic response behaviour of the electrode, but computational models can be and have been built which are nevertheless capable of making useful and very convincing predictions of the response behaviours exhibited by such a system. It is particularly noteworthy that such computational models have been very instrumental in driving an improved understanding of how the generated signal responses correlate with and are influenced by the physical properties of the system, enabling useful predictions to be made, and simultaneously influencing both the physical design of the working electrodes, and the selection of algorithms used to translate the response signals into results benefitting from improved accuracy, by virtue of having been corrected both for errors arising from manufacturing variation, and simultaneously for systematic errors arising from inherent differences arising within the sample composition itself, as described herein. Whilst such computational models been used to qualitatively validate the influences and effects described herein and thereby demonstrate the intrinsic utility of the approach being undertaken, it should be noted that the actual system is further complicated from this two domain model, in that the critical properties described in the model above which drive the signal response are actually prone to change themselves throughout the course of the measurement, as the reagent pad increasingly interacts with the bulk sample and *vice versa.*

The expected cascade of such interaction between reagent pad and bulk sample as time progresses is demonstrated schematically in Figure 4. The reagent layer first solvates from a dense layer, thence becoming a swollen and solvated diffuse layer and thereafter undergoes full dissolution to be almost fully dispersed into and equilibrated with the sample bulk. Differences in the progress through these stages lead to changes in the predicted time-based characteristic response under different conditions. Controlling the speed and extent of these transitions by means of electrode design and reagent composition formulations allows the resulting time-based characteristic responses to be used optimally for the purposes of measurement and correction.

In the initial stages of the reagent pad solvation (t1), the reagent pad sits largely intact. Its diffusivity will be relatively low. Its partitioning may in some circumstances be high, since compositional differences between the pad and bulk will be at their maximum so differences in porosity, solvency and suchlike will be exaggerated. As a result, the penetration of interfering substances at this stage of the reaction may be limited and, if the sample is blood, the exclusion of cellular components - for example erythrocytes - from the pad layer will be maximised. As a result, the sensitivity of the raw signal to, for example, different blood sample haematocrit values, will be at a minimum during this stage of the process.

The system will have had little chance to equilibrate at this point and so the effect of any variations and physical defects within the reagent pad arising as a result of variations in the manufacturing process will be similarly exaggerated. The effective thickness of the reagent pad at this point will be low to moderate and at its most variable. The flux will be governed largely from within or near to the pad layer.

As time progresses (t2) and more material exchange between the pad and the bulk occurs, the reagent pad will swell. Its diffusivity will increase, as will its thickness, initially. Its ability to partition will reduce. However defects and inconsistencies created within the reagent pad by the manufacturing process will have had an opportunity to equilibrate out somewhat, and improvements in signal consistency and precision between sensors will be observed. As reactants are consumed in the vicinity of the electrode, the concentration gradient dc/dx becomes shallower and the flux is governed by a balance of both the reagent pad and the bulk layer properties.

As time progresses further the reagent pad may reach a stage of almost complete disintegration. At this point, the pad has effectively equilibrated with the bulk sample. It no longer functions as a partition layer in any meaningful sense. The sensor effectively consists of a single bulk layer adjacent to the electrode ($x \rightarrow 0$) with a diffusivity controlled almost completely by the bulk sample properties, with the effect of the reagent pad properties becoming negligible. At this point, the effect of manufacturing variations within the reagent pad on the response will have been minimised and the optimal signal consistency and precision between sensors will be observed.

However, at this stage, inaccuracies arising from systematic differences arising within the sample itself e.g. due to the presence of interfering substances or due to the presence of cellular components affecting the diffusion processes through which the response signal is derived, will be correspondingly maximised.

Through understanding these processes, a trade off becomes apparent between how best to design the sensor with respect to how and when it should transition through these stages and what measurement and correction information becomes available within the response signal at different time points corresponding to these stages, and how best this information might be utilised.

Specifically, formulating and designing a sensor pad that (within the analyte test assay time) rapidly equilibrates and moves quickly towards the full disintegration stage at $t_3$ will maximise precision and reduce imprecision errors arising from manufacturing variation. However, such a system will be prone to suffer unduly from systematic inaccuracies between samples arising from variations in the diffusional and solvency properties of the bulk sample containing the analyte. For example, where the sample is blood, differences will arise from variations in the packed red blood cell volume.

Conversely, formulating and designing a sensor pad that (within the analyte test assay time) moves slowly through stages t, and $t_2$ will reduce the effects arising from variations in the diffusional and solvency properties of the bulk sample containing the analyte - for example, differences arising from variations in the packed red blood cell volume where the sample is blood, at some time points, whilst at the same time potentially maximising the information available at these different time points by which such effects could be corrected for algorithmically. However, such a formulation will suffer from increased imprecision caused by manufacturing variations in the reagent pad that have not had adequate opportunity to equilibrate out.

Example expected signal responses from systems designed with different pad thicknesses and compositions which influence diffusivity and equilibration time are shown in Figures 5a-5d.

Providing a device with first and second analyte reagents that are deliberately configured to operate at a different extreme in terms of the transitioning described above maximises both the quantity and quality of totally available information with which to extract a measurement. This enables - by means of a suitable algorithm - a more accurate measurement which thereby suffers to a lesser extent from errors arising from variations in the diffusional and solvency properties of the bulk sample containing the analyte (for example differences arising from variations in the packed red blood cell volume in situations where the sample is blood), while at the same time suffering to a lesser extent from imprecision errors caused by manufacturing variations in the analyte reagent that have not had adequate opportunity to equilibrate out.

The invention will be described further in the following non-limiting illustrative examples. However, it should be noted that whilst signal differences arising from changes in the relative exclusion of erythrocytes (i.e. Haematocrit) is cited as a significant example of how such a differential electrode and algorithmic approach can be used to derive insights and information through which inaccuracies may be corrected, it will also be apparent to those skilled in the art that other interfering effects - such as - for example- direct electrochemical interferents - will also be subject to such differential partitioning depending on how the reagent pads are configured and how they are designed to react over time, and therefore this invention also readily lends itself to other such additional error correction strategies.

**Example 1**

An enzyme ink formulation for an analyte measurement working electrode with rapid dissolution, diffusional exchange and equilibration with the bulk sample was formulated as follows and is designated 'INK A'.

100ml of 0.1mol/L Potassium Phosphate buffer aqueous solution adjusted to pH 7.4 was prepared.

3g of Hydroxyethyl Cellulose grade 250MBR (Ashland Chemicals Ltd) was added and dissolved to a viscous paste.

To this the following were added:

1.5 g of fumed silica (HDK K40 grade, from Wacker GmbH)
0.5 g of Tergitol NP/9 Surfactant (Sigma) (Density = 1.055g/mL)
Surface tension = 32 (dynes/cm at 1% actives, 25°C)
Draves wetting = 0.06 (draves 20 sec wetting conc, wt% at 25°C)
21s at 0.05wt% at 25°C)
2.0g of Glucose Dehydrogenase FADH enzyme (Sekisui Diagnostics)
15.0g Potassium hexacyanoferrate (III) (Fisher Scientific)

The components were mixed to a homogenous paste.

An electrochemical test strip was manufactured by screen printing conductive carbon ink onto a substrate into a defined pattern, and optionally over-coating this with a dielectric layer to expose defined working electrode areas, counter electrode areas and electrical contacts. 2 layers of INK A above were screen-printed and dried onto one of the Working electrode areas and the Counter electrode area of the test strip, immediately prior to the lamination step described in example 1. After coating the electrode areas with INK A, a defined sample void volume was formed thereupon by laminating a suitable spacer layer and hydrophilic top layer onto the strip, such that sample fill openings and air escape openings are in operative fluid contact with the sample void.

It will be subsequently become apparent to those skilled in the art that INK A possesses the following critical properties, relative to INKS B and C below:

- a relatively low proportion of non-volatile components to solvent (water), leading to the formation of relatively thin layers upon drying which can therefore resolvate rapidly upon use
- a relatively high proportion of rapidly dissolving highly soluble inorganic species (potassium hexacyanoferrate (III)) compared to the amount of polymeric film former and binder. This will drive rapid solvation and equilibration with the bulk sample in use, and relatively rapid disintegration of the reagent layer.

- the high loading of highly soluble inorganic component potassium hexacyanoferrate (III) relative to the other components promotes crystalline growth of domains of pure potassium hexacyanoferrate (III) within the film upon drying of the ink, leading to heterogeneity of the film and producing relatively large domains of exceptionally rapid dissolution and fast diffusivity within the reagent pad layer. This crystallisation also creates a very high surface area in the dried layer, which further facilitates rapid dissolution of the reagent pad layer upon use.
- the relatively high level of non-film formers (insoluble silica and soluble potassium hexacyanoferrate (III)) to film forming materials and polymers (HEC & Surfactants) creates a more porous open membrane like structure within the reagent pad, (as opposed to a contiguous flat, dense film) which helps to drive the rapid dissolution and relatively high diffusivity of reagent pads composed of this and similar compositions.

Overall, a rapidly dissolving, high diffusivity reagent pad with little or limited propensity to differentially partition sub components of the bulk sample is the net result of such formulations and property selection.

It will be apparent to those skilled in the art that in the specific formulation A above the potassium hexacyanoferrate (III) is taking on the role of electrochemical mediator, shuttling electrons between enzyme and electrode within the redox reaction. It will also be apparent to those skilled in the art that the potassium hexacyanoferrate (III), at this high proportion relative to other ingredients, specifically, the film formers therein, is generating relatively large crystalline domains of rapid solvation that disrupt the formation of contiguous films which would otherwise occur, and assisting the rapid solvation and ultimate disintegration of the reagent layer thus formed in use. These two functions can be thought of as separate design elements that happen to coincide conveniently within a single material choice in this specific example. Alternative ingredient combinations can be conceived in which the desired role of electrochemical mediator and the desired role of disruption of the contiguous film formation may be achieved by a combination of separate ingredients, rather than by means of a single material assuming both functions simultaneously.

**Example 2**

An enzyme ink formulation for an analyte measurement working electrode with more progressive dissolution, diffusional exchange and equilibration with bulk sample was formulated as follows, and is designated 'INK B'.

100ml of 0.1mol/L Potassium Phosphate buffer aqueous solution adjusted to pH 7.4 was prepared.

15g of Hydroxyethyl Cellulose grade 250LR (Ashland Chemicals Ltd) was added and dissolved to a viscous paste with gentle agitation over 2 hours.

To this the following were added:

1.5 g of fumed silica (HDK K40 grade, from Wacker GmbH)
0.5 g of Tergitol NP/9 Surfactant (Sigma)
2.0g of Glucose Dehydrogenase FADH enzyme (Sekisui Diagnostics)
15.0g Potassium hexacyanoferrate (III) (Fisher Scientific)

The components were mixed to a homogenous paste.

2 layers of INK B above were screen-printed and dried onto one of the Working electrode areas of a test strip as described in Example 1.

**Example 3**

An enzyme ink formulation for an analyte measurement working electrode with an even more progressive dissolution, diffusional exchange and equilibration with bulk sample was formulated as follows and is designated 'INK C'.

100ml of 0.1mol/L Potassium Phosphate buffer aqueous solution adjusted to pH 7.4 was prepared.

20g of Hydroxyethyl Cellulose grade 250LR (Ashland Chemicals Ltd) was added and dissolved to a viscous paste with gentle agitation over 2 hours.

To this the following were added:

1.5 g of fumed silica (HDK K40 grade, from Wacker GmbH)
0.5 g of Tergitol NP/9 Surfactant (Sigma)
2.0g of Glucose Dehydrogenase FADH enzyme (Sekisui Diagnostics)
5.0g Potassium hexacyanoferrate (III) (Fisher Scientific)

The components were mixed to a homogenous paste.

2 layers of INK C above were screen-printed and dried onto one of the Working electrode areas of a test strips as described in Example 1.

It will be apparent to those skilled in the art that INK C below possesses the following critical properties, relative to INK A above:

- a relatively high proportion of non-volatile components to solvent (water), leading to the formation of relatively thick layers upon drying which will therefore resolvate less rapidly upon use.
- a relatively low proportion of rapidly dissolving highly soluble inorganic species (potassium hexacyanoferrate (III)) compared to the amount of polymeric film former, binder and surfactant. This will drive slower solvation and equilibration with the bulk sample in use, and less propensity for disintegration of the reagent pad layer in use.
- the relatively low loading of highly soluble inorganic species potassium hexacyanoferrate (III) relative to the other components inhibits crystalline growth of domains of pure Potassium hexacyanoferrate (III) within the film upon drying of the ink, leading to a relative absence of large domains of exceptionally rapid dissolution and fast diffusivity within the reagent pad layer. The relative absence of such crystallisation also creates a more homogeneous film with lower surface area in the dried layer, which facilitates less rapid dissolution of the reagent pad layer upon use.
- the relatively low level of non-film formers (silica and potassium hexacyanoferrate (III)) relative to film forming polymer (HEC) creates a more dense, closed membrane like structure within the reagent pad, which helps to limit the dissolution and diffusivity of reagent pads composed of this and similar compositions, and increases the propensity for selective partitioning of some sample sub-components therein.

Overall, a progressively dissolving, lower diffusivity reagent pad with some initial propensity to differentially partition sub components of the bulk sample into the reagent pad - at least initially - is the net result of such formulations and property selection.

**Example 4**

Test strips were prepared using the process described in example 1 with a reagent layer comprising INK A coated onto one of the working electrodes and the counter electrode, and with INK B coated onto an additional adjacent working electrode.

**Example 5**

Test strips were prepared using the process described in example 1 with a reagent layer comprising INK A coated onto one of the working electrodes and the counter electrode, and with INK C coated onto an additional adjacent working electrode.

**Example 6**

Single use disposable electrochemical test strips for the measurement of glucose within whole blood were prepared using INK A only, according to the process described in Example 1.

These strips were then tested using multiple samples of whole blood containing different concentrations of beta-D-Glucose analyte varying between approximately 50mg/dL and ~550mg/dl with 3 additional intermediate levels included, and in which the Packed Cell Volume (Haematocrit) was varied between 20% v/v and 60%v/v, with additionally 3 intermediate haematocrit levels included. An applied potential of 300mV was set between the working electrode and the counter electrode on the strip, before sample was introduced and the resulting current measured over a time of approximately 10 seconds.

Selected examples of the current signal response profiles produced are shown in Figures 6a and 6b.

Figure 6a shows an example of the time-based response characteristics observed when multiple near identical electrodes with the same reagent formulation were exposed to similar samples with different concentrations of analyte, to produce a response whose magnitude could readily be used to effect a measurement of the analyte concentration by means of a suitable algorithm. In this regard, reference is made to ISO15197. It is apparent that the sensor response signal is influenced not only by the concentration of the analyte of interest, but also by systematic differences and variations within the diffusional and solvency properties of the bulk sample volume containing the analyte, of which the packed red blood cell volume or 'Haematocrit' is just one such example.

**Example 7**

Single use disposable test strips were prepared using the process described in Example 1 with a reagent layer comprising ink A from Example 1 coated onto one of the working electrodes (working electrode 1 - abbreviated we1 subsequently) and the counter electrode, and with ink B from example 2 coated onto an additional adjacent working electrode (working electrode 2, abbreviated we2 subsequently). These electrodes were then tested in an equivalent fashion to those described in Example 6 above, but with both electrodes being polarised identically relative to the counter electrode, and with the resulting current responses being measured through each.

**Measurement Algorithm for Combined Electrode Outputs**

In the embodiment, a new algorithm was employed to make an enhanced measurement based upon the combination of both signal responses from both electrodes. By exploiting the intentionally designed different time-based response characteristics of the different analyte reagents, an enhanced balance of precision and haematocrit insensitivity in the final measured response may be achieved that would not have been possible with either electrode system taken in isolation or if the different time-based response characteristics were not present.

In this embodiment, the first and second electrodes are polarised with respect to a counter/reference electrode. One or more first currents $i_{we1}$ are measured at the first working electrode over a first time period $[t_k; t_{k+n}]$, and one or more second currents $i_{we2}$ are measured at the second working electrode over second and third time periods $[t_a; t_b]$ and $[t_c; t_d]$. The one or more first and second currents are used to determine a corrected current measurement according to the following algorithm.

The mean of the first currents on the first working electrode between time k and time k+n is determined according to:

$$I_{1(actual)} = \frac{1}{n}\sum_{j=k}^{k+n} i_{we1}(t_j)$$

The sum $Q_1$ of the second currents on the second working electrode between $t_a$ and $t_b$ is determined according to:

$$Q_1 = \sum_{j=a}^{b} i_{we2}(t_j)$$

The sum $Q_2$ of the second currents on the second working electrode between $t_c$ and $t_d$ is determined according to:

$$Q_2 = \sum_{j=c}^{d} i_{we2}(t_j)$$

$Q_\Delta$ is determined according to:

$$Q_\Delta = Q_1 - Q_2$$

A predicted current on the first working electrode based on ($Q_\Delta$) is determined according to:

$$I_{1(predicted)} = \beta_0 + \beta_1 Q_\Delta$$

A deviation of the actual current at the first working electrode is determined according to:

$$I_\Delta = I_{1(actual)} - I_{1(predicted)}$$

The deviation is checked to see if it is below a lower threshold:

$$|I_\Delta| < l_a$$

If the deviation is above higher threshold, then an error may be reported:

$$|I_{\Delta}| > h_a \rightarrow \quad Report\ an\ Error$$

Otherwise:

$$l_a <= |I_{\Delta}| <= h_a$$

The corrected current is a mean of the actual and predicted currents:

$$I_{corr} = \overline{I_{1(actual)}, I_{1(predicted)}}$$

A derived haematocrit prediction is based on the deviation and the predicted current:

$$H_{predicted} = \beta_2 + \beta_3 I_{1(predicted)} + \beta_4 I_{\Delta}$$

A derived haematocrit deviation is determined according to:

$$H_{\Delta} = 42 - H_{predicted}$$

The corrected current is derived from the haematocrit deviation:

$$I_{corr} = \frac{I_{1(actual)}(100 - H_{\Delta} \cdot h_b)}{100 w_f}$$

With a weighting function such that:

$$I_{1(actual)} < I_{nominal}$$

$$w_f = \frac{I_{1(actual)}}{I_{nominal}}$$

$$I_{1(actual)} >= I_{nominal}$$

$$w_f = 1$$

The below coefficients were used in this example:

| Coefficient | Description | Value |
|---|---|---|
| k | Time in seconds | 5.50 |
| n | Time in seconds | 2.00 |
| a | Time in seconds | 2.50 |
| b | Time in seconds | 4.00 |
| c | Time in seconds | 5.50 |
| d | Time in seconds | 7.70 |
| $\beta_0$ | Current scaling term | 0.3027 $\mu$Amps |
| $\beta_1$ | Current scaling term | 8.72e-8 Amps |

(continued)

| Coefficient | Description | Value |
|---|---|---|
| $l_a$ | Low correction current threshold | 0.0800 μAmps |
| $h_a$ | High correction current cut off | 2e-6 Amps |
| $\beta_2$ | Hematocrit scaling term | 25.07 |
| $\beta_3$ | Hematocrit scaling term | 1.485e7 |
| $\beta_4$ | Hematocrit scaling term | 3.578e7 |
| $h_b$ | Hematocrit compensation term | 1.5 |
| $I_{nominal}$ | Nominal current for weighting | 1e-6 Amps |

$I_{corr}$ is converted to an estimate of sample concentration as per the following:

$$c = p_1 I_{corr} + p_2$$

$p_1$ and $p_2$ are calibration coefficients that can be readily empirically derived by means of data regression using suitable methods (e.g. linear least squares regression) and then applied predictively to subsequent equivalent test cases for the purposes of measurements. In the present embodiment, $p_1$ is 192 and $p_2$ is -27. It will also be apparent that the algorithm coefficient values arrived at above are a function of the specific electrode design and applied potential employed, and may also be arrived at empirically by means of data regression using suitable methods (e.g. linear least squares regression as an example).

The coefficients $\beta_0$ and $\beta_1$ are most easily determined empirically by linear regression. When the system design and formulations are fixed then the relationship between $Q_\Delta$ and $I_{1actual}$ at nominal haematocrit also (in theory) becomes fixed (leaving aside noise/error) for any set of time points. Therefore, testing strips with different glucose concentrations at a nominal, fixed haematocrit, and measuring the $h_{actual}$ and $Q_\Delta$ for these will elucidate the coefficients by which they are related. $\beta_0$ and $\beta_1$ can be used subsequently to derive $I_{1pred}$ values. Where the haematocrit is nominal, $I_{1pred}$ should approximate to $I_{1actual}$. Where the haematocrit deviates from nominal, $I_\Delta$ is non-zero and betrays the haematocrit information which has been uncovered by the differential design.

The table below quantifies the magnitude of the imprecision which was observed in this example, comparing the measured response arising from the ink A electrode only, from the ink B electrode only, and from the algorithmic combination of both electrodes, expressed as the standard deviation arising from multiple repeat measurements when calculated as a percentage of the reference measurement reading at that level. The precision measurement at each of the 5 haematocrit levels is shown together with the overall standard deviation encompassing all of the separate haematocrit levels.

The table also shows, for each of the 3 cases, the systematic error that arises from and is attributable to changes in sample haematocrit, expressed as the percent change in measured response that is directly attributable to every 1 percent change in haematocrit, with negative values indicating a declining response with increasing haematocrit, and positive values indicating an increasing response with increasing haematocrit.

| | HCT% | INK B | INK A | Algorithm | |
|---|---|---|---|---|---|
| | 20.00 | 7.84 | 4.83 | 8.87 | %sd |
| | 29.50 | 8.82 | 5.28 | 9.67 | %sd |
| | 43.80 | 17.56 | 6.75 | 12.64 | %sd |
| | 50.40 | 18.34 | 6.32 | 16.52 | %sd |
| | 59.40 | 20.28 | 8.64 | 17.34 | %sd |
| **mean std (Imprecision)** | | **14.56** | **6.36** | **13.01** | |
| **Mean Hct slope (error) %/%** | | **-0.98** | **-1.24** | **-0.19** | |

It can be readily seen from this table that this combined system demonstrates a reduced haematocrit sensitivity when compared with the ink A based measurement used in isolation, and also gives improved precision, when compared with the ink B based measurement when this is used in isolation.

**Example 8**

Single use disposable test strips were prepared using the process described in example 1 with a reagent layer comprising ink A from example 1 coated onto one of the working electrodes (working electrode 1 - abbreviated we1 subsequently) and the counter electrode, and with ink C from example 3 coated onto an additional adjacent working electrode (working electrode 2, abbreviated we2 subsequently).

An algorithm of the type described in example 7, making an enhanced measurement based upon the combination of both signal responses from both electrodes, specifically exploiting the different time-based response characteristic of each ink, was used to generate measured responses as described therein.

In this instance, the equations used were as above and the algorithm coefficients utilised were as follows:

| Coefficient | description | Value |
|---|---|---|
| k | Time in seconds | 5.50 |
| n | Time in seconds | 2.00 |
| a | Time in seconds | 2.0 |
| b | Time in seconds | 3.5 |
| c | Time in seconds | 5.50 |
| d | Time in seconds | 7.70 |
| $\beta_0$ | Current scaling term | 0.64 gamps |
| $\beta_1$ | Current scaling term | 9.98e-8 Amps |
| $l_a$ | Low correction current threshold | 0.120 $\mu$Amps |
| $h_a$ | High correction current cut off | 2e-6 Amps |
| $\beta_2$ | Hematocrit scaling term | 20.1 |
| $\beta_3$ | Hematocrit scaling term | 1.7e7 |
| $\beta_4$ | Hematocrit scaling term | 3.2e7 |
| $h_b$ | Hematocrit compensation term | 1.8 |
| $I_{nominal}$ | Nominal current for weighting | 1e-6 Amps |
| $P_1$ | Coefficient term (slope) | 259 |
| $P_2$ | Coefficient term (intercept) | -60.7 |

The table below quantifies the magnitude of the imprecision which was observed from comparing the measured response arising from the ink A electrode only (we1), from the ink C electrode only (we2) and from the algorithmic combination of both electrodes, as well as the systematic error that is attributable to changes in sample haematocrit in each case, as described in example 6 above.

| | **HCT %** | **ink A** | **ink C** | **algorithm** | |
|---|---|---|---|---|---|
| | 19.40 | 8.15 | 16.79 | 17.89 | %sd |
| | 29.70 | 6.96 | 16.29 | 13.93 | %sd |
| | 41.40 | 8.90 | 30.36 | 11.85 | %sd |
| | 49.80 | 16.19 | 14.10 | 18.63 | %sd |
| | 59.10 | 16.38 | 21.65 | 21.67 | %sd |
| **Mean std (imprecision)** | | **11.32** | **19.84** | **16.80** | |
| **Hct effect %/% (error)** | | **-1.24** | **-0.66** | **0.17** | |

**Example 9** - not according to the present invention

Single use disposable test strips were prepared using the process described in example 1 with a reagent layer comprising ink A from example 1 coated onto one of the working electrodes (working electrode 1 - abbreviated we1 subsequently) and the counter electrode, and with ink C from example 3 coated onto an additional adjacent working electrode (working electrode 2, abbreviated we2 subsequently).

**Algorithm Development Procedure**

An algorithm not according to the present invention was used which employs the combined response from both electrodes taken at multiple time points.

An outline of the process by which such an algorithm can be arrived at and subsequently used is shown schematically in Figure 7.

In this specific example, current measurement in amps were taken for Ink C (we2) at 1 second, 2 seconds, 3 seconds, 4 seconds, 5 seconds, 6 seconds, 7 seconds and 8 seconds, and for Ink A (we1) at 1 second, 2 seconds, 3 seconds, 4 seconds, 5 seconds, 6 seconds, 7 seconds and 8 seconds, to create a total of 16 signal inputs for each measurement. These inputs were presented in the order listed here, and numbered 1 to 16.

This algorithm employed the methodology of Principal Components Analysis (PCA) to orthogonalise the variation between all 16 of these inputs and used that variation to produce models with enhanced predictive capability.

In this instance, the raw inputs were first translated into a 'z-score' by subtracting the nominal mean and nominal standard deviation from each response: values used are mean and standard deviation of all readings expected from across the full range of analyte concentrations and haematocrits from which measurements would be taken, for each time point and from each electrode.

The resulting 16 inputs were then translated to their principle component scores using matrix multiplication using a coefficient matrix derived computationally by regression.

Actual values of the nominal mean and nominal standard deviations used for the z-score translations in this example, and the coefficient matrix used to convert the 16 raw inputs into their principal components in this instance are shown in Figure 8.

Each of the 16 resulting principle components that result from this matrix multiplication of the z scores with the coefficient matrix above was assigned a corresponding name x1 ,x2,x3...x16 and the model terms that were arrived at and used to predict the final responses, along with their coefficient values, are shown below: (with x1 being the coefficient associated with model term x1, and x1:x2 being the coefficient for the interaction between x1 and x2, i.e. the coefficient of their product).

| | |
|---|---|
| '(Intercept)' | 223.6234 |
| 'x1' | 40.45865 |
| 'x2' | 30.98085 |
| 'x3' | -21.3562 |
| 'x4' | 54.80837 |
| 'x5' | 3.584576 |
| 'x6' | 185.4266 |
| 'x7' | 79.81192 |
| 'x8' | -38.7733 |
| 'x9' | 67.59421 |
| 'x10' | 68.29765 |
| 'x11' | -142.966 |
| 'x12' | 237.6735 |
| 'x13' | 10.67812 |
| 'x14' | -348.413 |
| 'x15' | -74.6425 |
| 'x16' | -341.656 |
| 'x1:x2' | 10.45586 |
| 'x1:x3' | -1.05905 |
| 'x1:x4' | 15.6545 |
| 'x1:x5' | 6.383928 |

(continued)

| | |
|---|---|
| 'x1:x6' | 26.79171 |
| 'x1:x7' | 16.3628 |
| 'x1:x11' | -27.2193 |
| 'x1:x12' | 44.441 |
| 'x1:x14' | -87.5796 |
| 'x1 :x16' | -110.294 |
| 'x2:x3' | -29.0012 |
| 'x2:x5' | 48.27995 |
| 'x2:x12' | 226.5727 |
| 'x2:x14' | -680.894 |
| 'x3:x4' | -58.7888 |
| 'x3:x12' | -483.608 |
| 'x3:x15' | -1128.87 |
| 'x5:x7' | 623.4364 |
| 'x5:x8' | -1353.11 |
| 'x5:x14' | -3717.53 |
| 'x6:x8' | 2191.948 |
| 'x6:x10' | -1894.92 |
| 'x7:x11' | 2705.985 |
| 'x7:x14' | 10287.66 |
| 'x7:x15' | -10675.9 |
| 'x7:x16' | 11830.55 |
| 'x8:x16' | 20378.1 |
| 'x9:x12' | 4441.246 |
| 'x12:x16' | 15446.43 |
| 'x13:x14' | 26812.78 |

When this algorithm was applied to measurement data from the test strips described, with samples at 5 haematocrits (varying from 20% to 60%) and at 5 glucose levels (varying between 50 and 500 mg/dl), the responses which resulted are shown in Figure X.

It can be readily be seen through reference to the table below that this combined system demonstrates a reduced haematocrit sensitivity when compared with both the ink A and ink C based measurement used in isolation, and also gives improved precision, when compared with the ink C based measurement when this is used in isolation.

| %Hct | INK A | INK C | algorithm | |
|---|---|---|---|---|
| **20** | 11.24 | 27.48 | 9.07 | %sd |
| **29.5** | 5.38 | 19.39 | 8.44 | %sd |
| **43.8** | 6.16 | 10.52 | 10.25 | %sd |
| **50.4** | 8.72 | 12.57 | 7.50 | %sd |
| **59.4** | 9.81 | 18.51 | 11.68 | %sd |
| mean sd% (imprecision) | **8.26** | **17.70** | **9.39** | |
| Hct error %/% | **-1.31** | **-0.72** | **-0.06** | |

**Example** - not according to the present invention

Single use disposable test strips were prepared using the process described in example 1 with a reagent layer comprising ink A from example 1 coated onto one of the working electrodes (working electrode 1 - abbreviated we1 subsequently) and the counter electrode, and with ink C from Example 3 coated onto an additional adjacent working electrode (working electrode 2, abbreviated we2 subsequently).

In this instance a different electrochemical test procedure was used with a potential of +300mV applied for 8s, followed by a potential of -300mV for 2s to drive a reverse electrochemical reaction for a period, giving rise to additional, amplified current signals, an example of which is shown below.

A PCA algorithm of a similar type to that described in Example 9 was developed by computational regression as described above.

In this instance the 21 time points from the 2 electrodes inputs were used as shown in the below table:

| input # | 1 | 2 | 3 | 4 | 5 | 6 | 7 | 8 | 9 | 10 | 11 | 12 | 13 | 14 | 15 | 16 | 17 | 18 | 19 | 20 | 21 |
|---|---|---|---|---|---|---|---|---|---|---|---|---|---|---|---|---|---|---|---|---|---|
| we | 2 | 2 | 2 | 2 | 2 | 2 | 2 | 2 | 2 | 2 | 2 | 2 | 2 | 1 | 1 | 1 | 1 | 1 | 1 | 1 | 1 |
| time/s | 1 | 2 | 3 | 4 | 5 | 6 | 7 | 8 | 8.2 | 8.6 | 9 | 9.4 | 9.8 | 6 | 7 | 8 | 8.2 | 8.6 | 9 | 9.4 | 9.8 |
| potential/mV | 300 | 300 | 300 | 300 | 300 | 300 | 300 | 300 | -300 | -300 | -300 | -300 | -300 | 300 | 300 | 300 | -300 | -300 | -300 | -300 | -300 |

Z-score values were calculated using the following parameters:

| input # | 1 | 2 | 3 | 4 | 5 | 6 | 7 | 8 | 9 | 10 | 11 | 12 | 13 | 14 | 15 | 16 | 17 | 18 | 19 | 20 | 2 |
|---|---|---|---|---|---|---|---|---|---|---|---|---|---|---|---|---|---|---|---|---|---|
| mean | 1.20E-06 | 6.92E-07 | 4.79E-07 | 3.96E-07 | 3.58E-07 | 3.38E-07 | 3.26E-07 | 3.18E-07 | -2.79E-06 | -8.27E-07 | -4.86E-07 | -3.26E-07 | -2.38E-07 | 1.16E-06 | 1.12E-06 | 1.08E-06 | -9.47E-06 | -5.99E-06 | -4.75E-06 | -4.01E-06 | -3.51E-( |
| sd | 2.36E-07 | 2.34E-07 | 2.01E-07 | 1.83E-07 | 1.74E-07 | 1.69E-07 | 1.67E-07 | 1.65E-07 | 1.48E-06 | 6.42E-07 | 4.27E-07 | 3.13E-07 | 2.43E-07 | 6.97E-07 | 6.69E-07 | 6.47E-07 | 3.42E-06 | 2.59E-06 | 2.18E-06 | 1.91E-06 | 1.71E-( |

The transformation matrix was used to derive the Principal Components is shown in a Figure 9.

Each PCA term was entered into the model, with x1 representing PCA1, x2 representing PCA 2, etc. The model coefficients used to produce the final glucose result were as follows:

| | |
|---|---|
| '(Intercept)' | 234.7475 |
| 'x1' | -38.3099 |
| 'x2' | -15.3283 |
| 'x3' | -0.58258 |
| 'x4' | -50.661 |
| 'x5' | -0.92856 |
| 'x6' | -14.6272 |
| 'x7' | 22.84533 |
| 'x8' | 38.67765 |
| 'x9' | -56.5108 |
| 'x10' | 59.33 |
| 'x11' | 19.78744 |
| 'x12' | 69.83339 |
| 'x13' | 91.31363 |
| 'x14' | 69.44026 |
| 'x15' | -25.5952 |
| 'x16' | 109.5115 |
| 'x17' | 40.77065 |
| 'x18' | -183.638 |
| 'x19' | -304.011 |
| 'x20' | 59.07489 |
| 'x21' | 61.78801 |
| 'x1:x4' | 5.117411 |
| 'x1:x5' | -5.26479 |
| 'x1:x7' | 2.928789 |
| 'x1:x8' | 8.003721 |
| 'x1:x14' | 24.65715 |
| 'x1:x19' | 75.32047 |
| 'x2:x5' | -8.89656 |
| 'x2:x6' | -12.2507 |
| 'x2:x8' | 24.29088 |
| 'x2:x9' | 48.52244 |
| 'x3:x4' | -13.1471 |
| 'x3:x5' | -4.89167 |
| 'x3:x6' | 37.0657 |

(continued)

| | |
|---|---|
| 'x3:x10' | 52.2028 |
| 'x3:x16' | 184.0183 |
| 'x3:x19' | 390.3804 |
| 'x4:x6' | 30.96216 |
| 'x4:x7' | 25.25195 |
| 'x4:x8' | 67.82782 |
| 'x4:x20' | -697.834 |
| 'x5:x6' | -26.6007 |
| 'x5:x8' | 117.2217 |
| 'x5:x14' | -151.546 |
| 'x5:x19' | -500.499 |
| 'x6:x8' | -123.11 |
| 'x6:x9' | 363.2323 |
| 'x6:x18' | -585.834 |
| 'x6:x19' | 1300.351 |
| 'x6:x21' | -2018.16 |
| 'x7:x12' | 360.4495 |
| 'x8:x10' | -579.733 |
| 'x9:x18' | -2950.58 |
| 'x10:x11' | 1472.222 |
| 'x10:x15' | -2117.28 |
| 'x10:x19' | 4262.937 |
| 'x12:x13' | -1302.24 |
| 'x15:x18' | 7688.505 |
| 'x16:x17' | -6367.99 |
| 'x16:x19' | 15489.48 |

The results seen in Figures 10a-10d were obtained when this algorithm was applied to the test strip output signals to predict the analyte response, from blood samples whose glucose concentration and haematocrit level had been manipulated to the reference ranges shown below.

It can be seen from the table below that a precise measurement that is insensitive to the interfering effect of haematocrit changes is the result of this electrode design and algorithm combination.

| | **HCT %** | **INK A** | **INK C** | **algorithm** | |
|---|---|---|---|---|---|
| | 19 | 5.10 | 18.62 | 4.44 | %sd |
| | 29.2 | 4.89 | 21.35 | 5.24 | %sd |
| | 41.6 | 5.31 | 19.28 | 4.03 | %sd |
| | 50 | 8.37 | 26.26 | 4.92 | %sd |
| | 60 | 8.80 | 26.60 | 4.02 | %sd |
| **std deviation** (imprecision) | | **6.49** | **22.42** | **4.53** | |
| **HCT %/%** (error) | | **-1.4** | **-0.65** | **-0.01** | |

Whilst the invention has been described in connection with preferred embodiments, it is to be understood that the invention is not limited to these embodiments, and that alterations, modifications, and variations of these embodiments may be carried out by the skilled person without departing from the scope of the invention which is defined by the appended claims.

## Claims

**1.** A method of using a test device for measuring an amount of analyte in a fluid sample, the analyte being glucose, the test device comprising:

a substrate having thereon a first analyte reagent and a second analyte reagent, the first and second analyte reagents comprising film-forming components and non-film-forming components, the first analyte reagent comprising a relatively high proportion of non-film-forming components to film-forming components relative to the proportion of non-film-forming components to film-forming components in the second analyte reagent, wherein the first and second analyte reagents are respectively first and second inks each comprising an enzyme and a mediator;
a first electrode comprising said first analyte reagent formulated to react with said analyte to generate a signal indicative of the presence or amount of analyte in a sample, the first analyte reagent having a first time-based response characteristic; and a second electrode comprising said second analyte reagent formulated to react with said analyte to generate a signal indicative of the presence or amount of analyte in the sample, the second analyte reagent having a second time-based response characteristic, and wherein the first and second time-based response characteristics comprise respectively different first and second diffusional and/or dissolutional response characteristics, the method comprising:

polarising the first and second electrodes;
applying the fluid sample to the test device;
measuring one or more first currents at the first electrode;
measuring one or more second currents at the second electrode; and
using the one or more first and second currents to determine a corrected current measurement, **characterized in that**:

the one or more first currents are measured over a first time period that begins when the first analyte reagent is substantially completely dissociated or dissolved within the fluid sample,
the one or more second currents are measured over a second time period and a third time period, wherein the second time period begins when the second analyte reagent begins to dissociate or dissolve within the fluid sample, and wherein the third time period begins when dissolution or dissociation of the second analyte reagent within the fluid sample is not complete, and

wherein using the one or more first and second currents to determine the corrected current measurement comprises:

(i) determining a first current mean average by averaging the one or more first currents;
(ii) calculating a difference, $Q_\Delta$, of a sum, $Q_1$, of the one or more second currents measured over the second time period and a sum, $Q_2$, of the one or more second currents measured over the third time period,
(iii) determining a predicted first current, $I_{1(predicted)}$, on the first electrode based on $Q_\Delta$ using a fixed relationship: $I_{1(predicted)} = \beta_0 + \beta_1 Q_\Delta$, where $\beta_0$ and $\beta_1$ are empirically determined coefficients defining said fixed relationship between $Q_\Delta$ and $I_{1(predicted)}$;
(iv) determining said corrected current measurement by taking a mean average of said first current mean average and the predicted first current; and
(v) determining the amount of the analyte in the fluid sample based on the corrected current measurement.

**2.** The method of claim 1, wherein the first time period begins when the reaction between the first analyte reagent and the analyte is in a substantially steady state.

**3.** The method of any of claims 1-2, wherein:
the second time period begins when the reaction between the second analyte reagent and the analyte is in a substantially non-steady state.

**4.** The method of any of claims 1-3, wherein the third time period begins when dissolution or dissociation of the second analyte reagent within the fluid sample is in a substantially steady state.

**5.** The method of claim 1, further comprising:
carrying out the method of claim 1 for each of multiple of said test devices, thereby obtaining a set of predicted first currents; and

adjusting each predicted first current based on a linear regression analysis of the set of predicted first currents.

## Patentansprüche

1. Verfahren zur Verwendung einer Testvorrichtung zur Messung einer Menge eines Analyten in einer Fluidprobe, wobei der Analyt Glukose ist, wobei die Testvorrichtung Folgendes umfasst:

   ein Substrat, auf dem sich ein erstes Analyt-Reagens und ein zweites Analyt-Reagens befinden, wobei das erste und das zweite Analyt-Reagens filmbildende Komponenten und nicht filmbildende Komponenten umfassen, wobei das erste Analyt-Reagens ein relativ hohes Verhältnis von nicht filmbildenden Komponenten zu filmbildenden Komponenten relativ zu dem Verhältnis von nicht filmbildenden Komponenten zu filmbildenden Komponenten in dem zweiten Analyt-Reagens umfasst, wobei das erste und das zweite Analyt-Reagens jeweils eine erste und eine zweite Tinte sind, die jeweils ein Enzym und einen Mediator umfassen;
   eine erste Elektrode, die das erste Analyt-Reagens umfasst, das so formuliert ist, dass es mit dem Analyten reagiert, um ein Signal zu erzeugen, das die Anwesenheit oder Menge des Analyten in einer Probe anzeigt, wobei das erste Analyt-Reagens eine erste zeitbasierte Reaktionscharakteristik aufweist; und eine zweite Elektrode, die das zweite Analyt-Reagens umfasst, das so formuliert ist, dass es mit dem Analyten reagiert, um ein Signal zu erzeugen, das die Anwesenheit oder Menge des Analyten in der Probe anzeigt, wobei das zweite Analyt-Reagens eine zweite zeitbasierte Reaktionscharakteristik aufweist, und wobei die erste und die zweite zeitbasierte Reaktionscharakteristik jeweils unterschiedliche erste und zweite Diffusions- und/oder Auflösungsreaktionscharakteristiken umfassen, wobei das Verfahren Folgendes umfasst:

      Polarisieren der ersten und zweiten Elektrode;
      Aufbringen der Fluidprobe in die Testvorrichtung;
      Messen eines oder mehrerer erster Ströme an der ersten Elektrode;
      Messen eines oder mehrerer zweiter Ströme an der zweiten Elektrode; und
      Verwenden des einen oder der mehreren ersten und zweiten Ströme zum Bestimmen einer korrigierten Strommessung, **dadurch gekennzeichnet, dass**:

         der eine oder die mehreren ersten Ströme über eine erste Zeitspanne gemessen werden, die beginnt, wenn das erste Analyt-Reagens im Wesentlichen vollständig in der Fluidprobe dissoziiert oder gelöst ist, der eine oder die mehreren zweiten Ströme über eine zweite Zeitspanne und eine dritte Zeitspanne gemessen werden, wobei die zweite Zeitspanne beginnt, wenn das zweite Analyt-Reagens in der Fluidprobe zu dissoziieren oder sich aufzulösen beginnt, und wobei die dritte Zeitspanne beginnt, wenn die Auflösung oder Dissoziation des zweiten Analyt-Reagens in der Fluidprobe nicht vollständig ist, und wobei das Verwenden des einen oder der mehreren ersten und zweiten Ströme zum Bestimmen der korrigierten Strommessung Folgendes umfasst:

            (i) Bestimmen eines ersten Strommittelwertes durch Mittelwertbildung des einen oder der mehreren ersten Ströme;
            (ii) Berechnen einer Differenz, $Q_\Delta$, einer Summe, $Q_1$, des einen oder der mehreren zweiten Ströme, die über die zweite Zeitspanne gemessen wurden, und einer Summe $Q_2$ des einen oder der mehreren zweiten Ströme, die über die dritte Zeitspanne gemessen wurden,
            (iii) Bestimmen eines vorhergesagten ersten Stroms, $I_{1(predicted)}$, an der ersten Elektrode basierend auf $Q_\Delta$ unter Verwendung einer festen Beziehung: $I_{1(predicted)} = \beta_0 + \beta_1 Q_\Delta$, wobei $\beta_0$ und $\beta_1$ empirisch bestimmte Koeffizienten sind, die die feste Beziehung zwischen $Q_\Delta$ und $I_{1(predicted)}$ definieren;
            (iv) Bestimmen der korrigierten Strommessung durch Bilden eines Mittelwerts aus dem ersten Strommittelwert und dem vorhergesagten ersten Strom; und
            (v) Bestimmen der Menge des Analyten in der Fluidprobe auf der Grundlage der korrigierten Strommessung,

2. Verfahren nach Anspruch 1, wobei die erste Zeitspanne beginnt, wenn sich die Reaktion zwischen dem ersten Analyt-Reagens und dem Analyten in einem im Wesentlichen stetigen Zustand befindet.

3. Verfahren nach einem der Ansprüche 1 bis 2, wobei:
   die zweite Zeitspanne beginnt, wenn sich die Reaktion zwischen dem zweiten Analyt-Reagens und dem Analyten in einem im Wesentlichen nicht-stetigen Zustand befindet.

**4.** Verfahren nach einem der Ansprüche 1 bis 3, wobei die dritte Zeitspanne beginnt, wenn sich die Auflösung oder Dissoziation des zweiten Analyt-Reagens innerhalb der Fluidprobe in einem im Wesentlichen stetigen Zustand befindet.

**5.** Verfahren nach Anspruch 1, ferner umfassend:

Ausführen des Verfahrens nach Anspruch 1 für jede der mehreren Testvorrichtungen, wodurch ein Satz vorhergesagter erster Ströme erhalten wird; und
Anpassen jedes vorhergesagten ersten Stroms auf der Grundlage einer linearen Regressionsanalyse des Satzes der vorhergesagten ersten Ströme,

## Revendications

**1.** Procédé d'utilisation d'un dispositif de test pour mesurer une quantité d'analyte dans un échantillon de fluide, l'analyte étant du glucose, le dispositif de test comprenant :

un substrat comportant sur celui-ci un premier réactif d'analyte et un deuxième réactif d'analyte, les premier et deuxième réactifs d'analyte comprenant des constituants filmogènes et des constituants non filmogènes, le premier réactif d'analyte comprenant une proportion relativement élevée de constituants non filmogènes par rapport aux constituants filmogènes, et par rapport à la proportion de constituants non filmogènes relativement aux constituants filmogènes dans le deuxième réactif d'analyte, dans lequel les premier et deuxième réactifs d'analyte sont respectivement des première et deuxième encres comprenant chacune un enzyme et un médiateur ;
une première électrode comprenant ledit premier réactif d'analyte formulé de façon à réagir avec ledit analyte pour générer un signal indiquant la présence ou la quantité d'analyte dans un échantillon, le premier réactif d'analyte ayant une première caractéristique de réponse temporelle, et une deuxième électrode comprenant ledit deuxième réactif d'analyte formulé de façon à réagir avec ledit analyte pour générer un signal indiquant la présence ou la quantité d'analyte dans l'échantillon, le deuxième réactif d'analyte ayant une deuxième caractéristique de réponse temporelle, et dans lequel les première et deuxième caractéristiques de réponse temporelles comprennent respectivement des première et deuxième caractéristiques de réponse de diffusion et/ou de dissolution différentes, le procédé comprenant :

la polarisation des première et deuxième électrodes ;
l'application de l'échantillon de fluide sur le dispositif d'essai ;
la mesure d'un ou plusieurs premiers courants au niveau de la première électrode ;
la mesure d'un ou plusieurs deuxièmes courants au niveau de la deuxième électrode ; et
l'utilisation des un ou plusieurs premiers et deuxièmes courants pour déterminer une mesure de courant corrigée, **caractérisé en ce que** :

les un ou plusieurs premiers courants sont mesurés au cours d'une première période de temps qui débute lorsque le premier réactif d'analyte est sensiblement complètement dissocié ou dissous dans l'échantillon de fluide,
les un ou plusieurs deuxièmes courants sont mesurés au cours d'une deuxième période de temps et d'une troisième période de temps, dans lequel la deuxième période de temps débute lorsque le deuxième réactif d'analyte commence à se dissocier ou se dissoudre dans l'échantillon de fluide, et dans lequel la troisième période de temps débute lorsque la dissolution ou dissociation du deuxième réactif d'analyte dans l'échantillon de fluide n'est pas complète, et
dans lequel l'utilisation des un ou plusieurs premiers et deuxièmes courants pour déterminer la mesure de courant corrigée comprend :

(i) la détermination d'un premier courant moyen en faisant la moyenne des un ou plusieurs premiers courants ;
(ii) le calcul d'une différence $Q_\Delta$ entre une somme $Q_1$ des un ou plusieurs deuxièmes courants mesurés au cours de la deuxième période de temps et une somme $Q_2$ des un ou plusieurs deuxièmes courants mesurés au cours de la troisième période de temps,
(iii) la détermination d'un premier courant prévu $I_{1(predicted)}$ sur la première électrode sur la base de $Q_\Delta$ en utilisant une relation fixe $I_{1(predicted)} = \beta_0 + \beta_1 Q_\Delta$, où $\beta_0$ et $\beta_1$ sont des coefficients déterminés empiriquement qui définissent ladite relation fixe entre $Q_{\Delta}$ et $I_{1(predicted)}$ ;
(iv) la détermination de ladite mesure de courant corrigée en prenant une moyenne du dit premier courant moyen et du premier courant prévu ; et
(v) la détermination de la quantité de l'analyte dans l'échantillon de fluide sur la base de la mesure de courant corrigée.

**2.** Procédé selon la revendication 1, dans lequel la première période de temps débute lorsque la réaction entre le premier réactif d'analyte et l'analyte est dans un état sensiblement stable.

**3.** Procédé selon l'une quelconque des revendications 1 à 2, dans lequel :
la deuxième période de temps débute lorsque la réaction entre le deuxième réactif d'analyte et l'analyte est dans un état sensiblement non stable.

**4.** Procédé selon l'une quelconque des revendications 1 à 3, dans lequel la troisième période de temps débute lorsque la dissolution ou dissociation du deuxième réactif d'analyte dans l'échantillon de fluide est dans un état sensiblement stable.

**5.** Procédé selon la revendication 1, comprenant en outre :

l'exécution du procédé selon la revendication 1 pour chacun desdits plusieurs dispositifs de test, pour obtenir ainsi une série de premiers courants prévus ; et
l'ajustement de chaque premier courant prévu sur la base d'une analyse de régression linéaire de la série de premiers courants prévus.

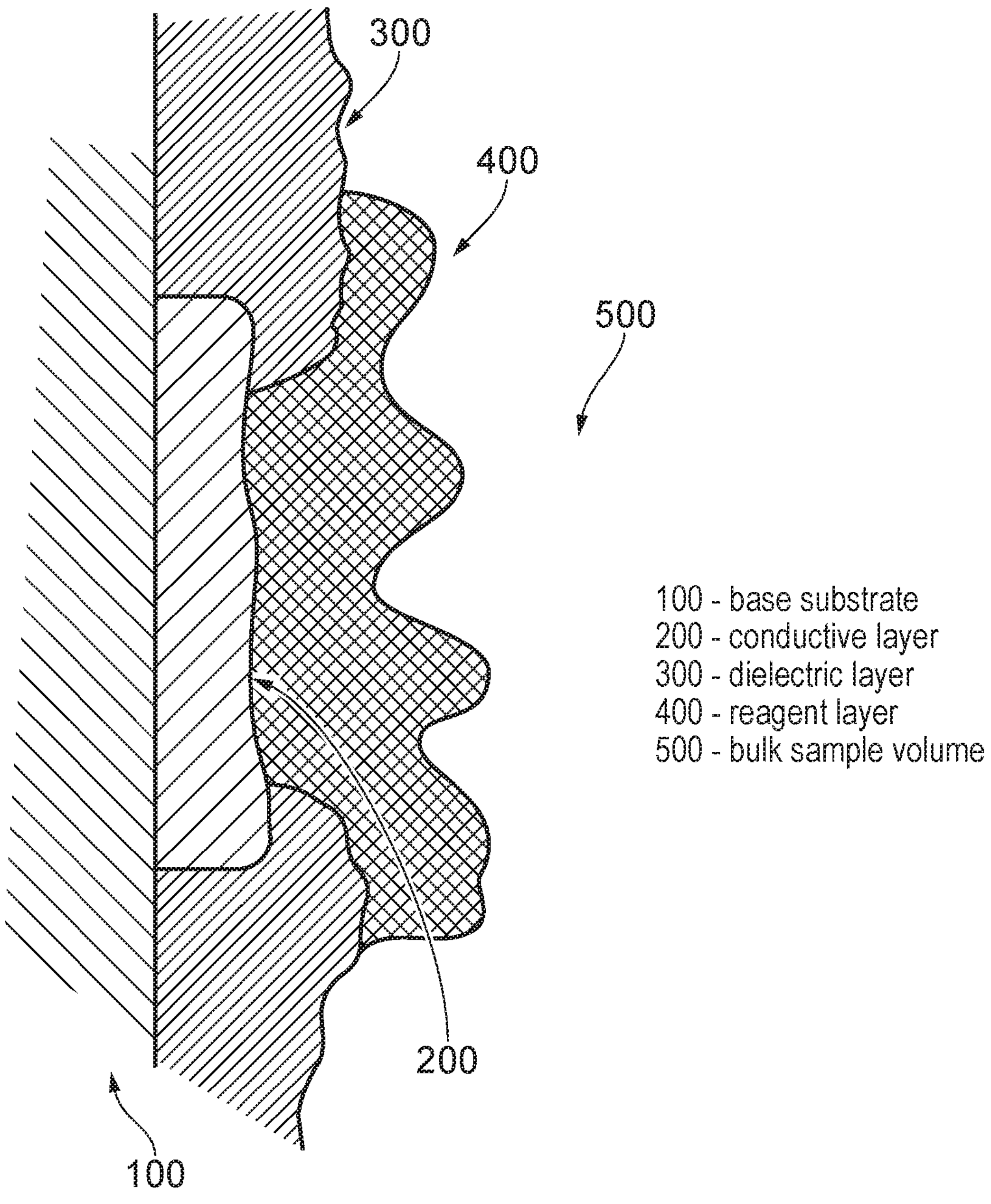
300
400
500
100 - base substrate
200 - conductive layer
300 - dielectric layer
400 - reagent layer
500 - bulk sample volume
200
100

FIG. 1

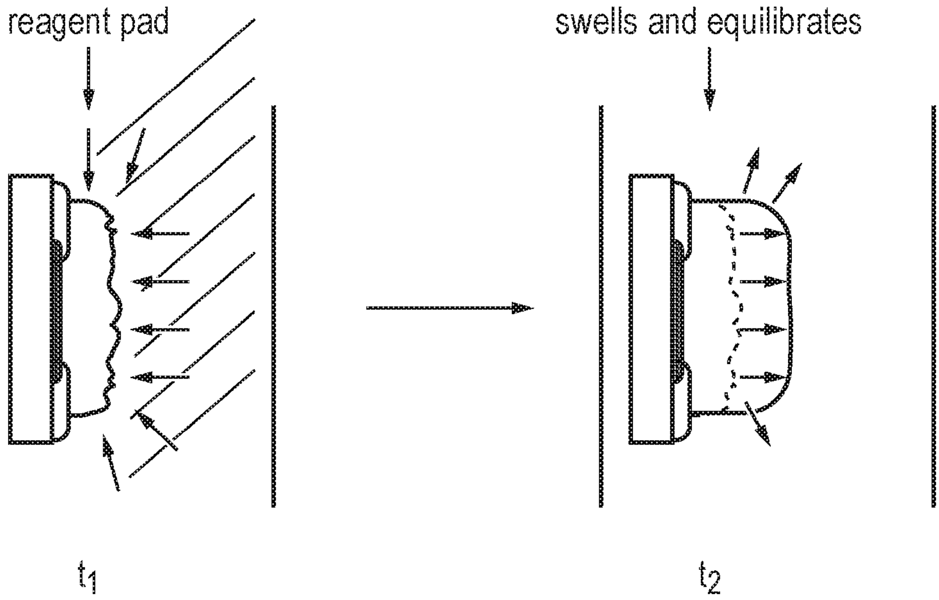
reagent pad
swells and equilibrates
t1
t2

FIG. 2

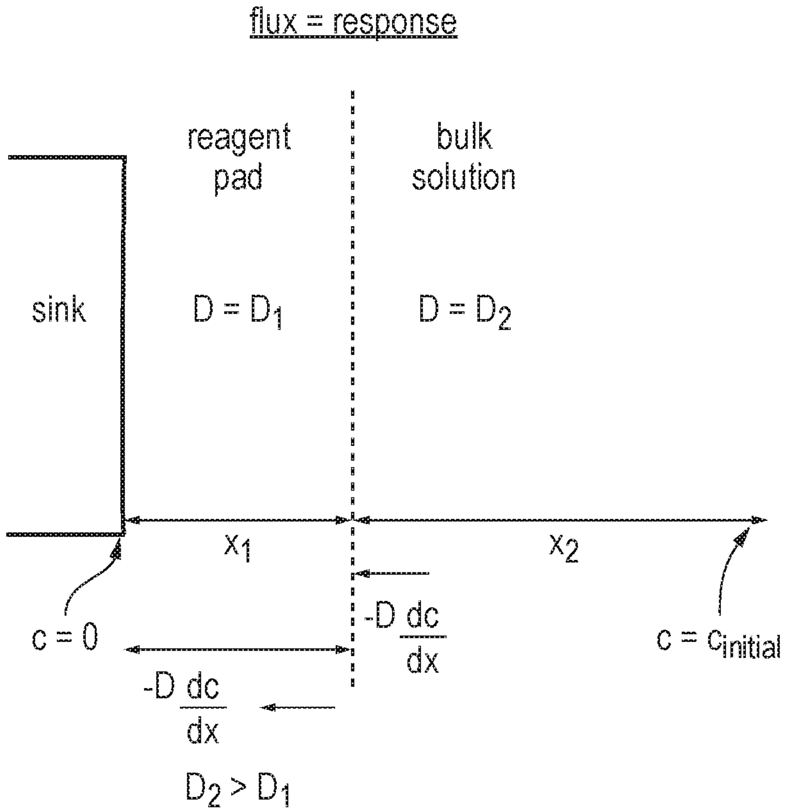
flux = response
reagent pad
bulk solution
sink
D = D1
D = D2
x1
x2
c = 0
-D dc/dx
-D dc/dx
c = c_initial
D2 > D1

FIG. 3

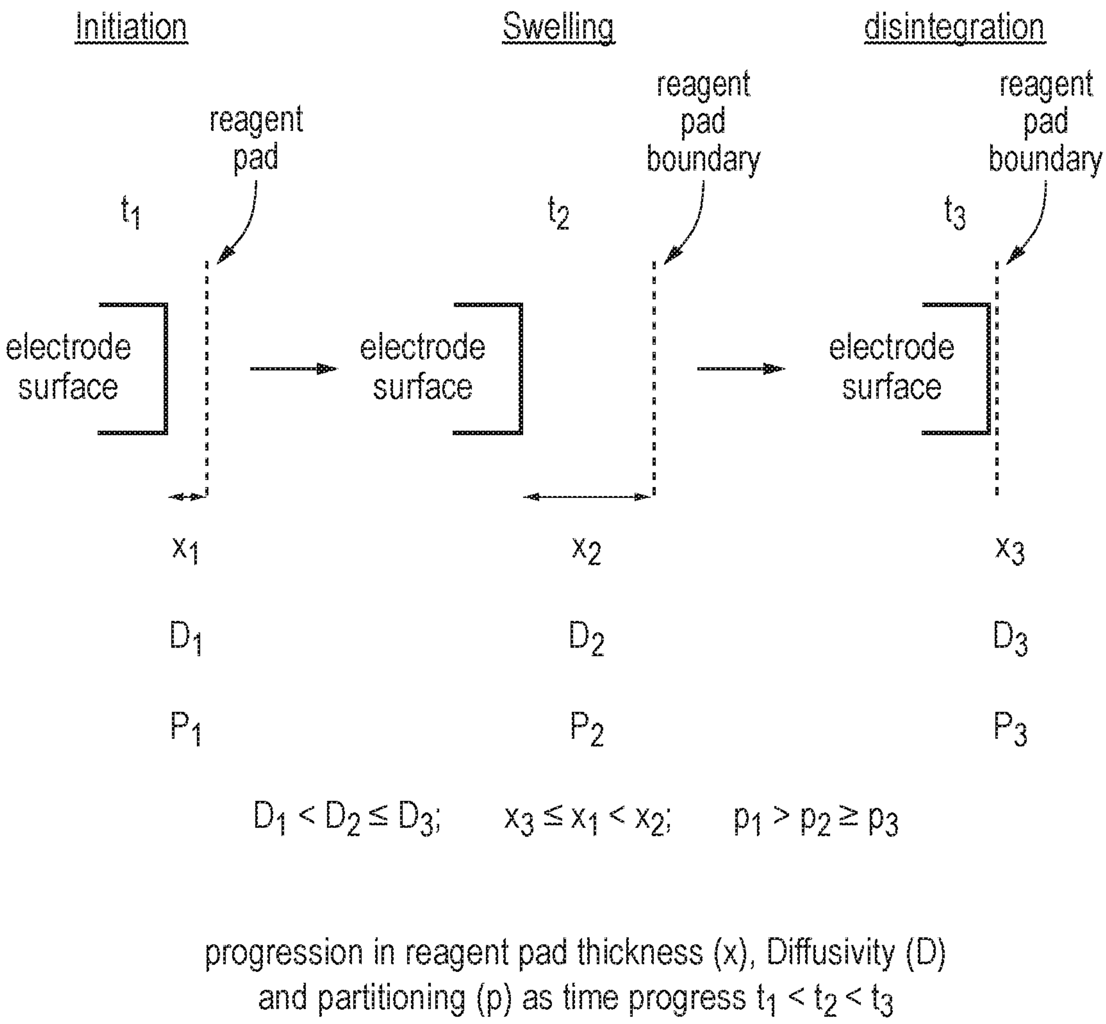
Initiation
Swelling
disintegration
reagent pad
reagent pad boundary
reagent pad boundary
$t_1$
$t_2$
$t_3$
electrode surface
electrode surface
electrode surface
$x_1$
$x_2$
$x_3$
$D_1$
$D_2$
$D_3$
$P_1$
$P_2$
$P_3$
$D_1 < D_2 \leq D_3$; $x_3 \leq x_1 < x_2$; $p_1 > p_2 \geq p_3$
progression in reagent pad thickness (x), Diffusivity (D) and partitioning (p) as time progress $t_1 < t_2 < t_3$

FIG. 4

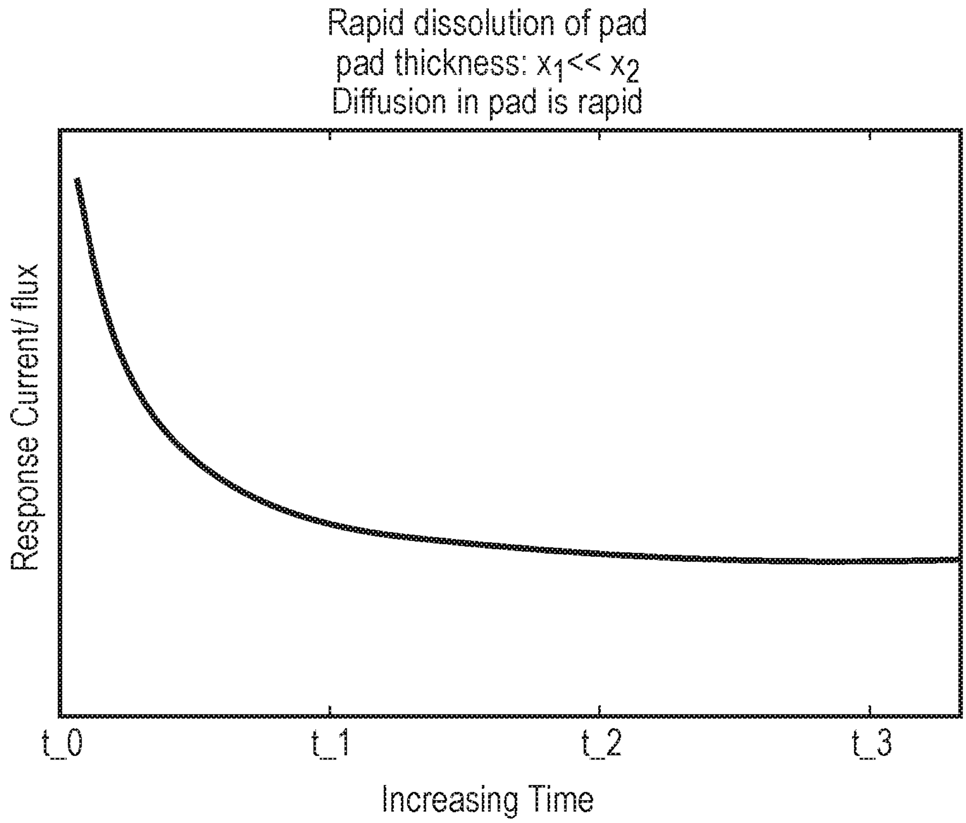
Rapid dissolution of pad
pad thickness: x1<< x2
Diffusion in pad is rapid
Response Current/ flux
t_0
t_1
t_2
t_3
Increasing Time

FIG. 5a

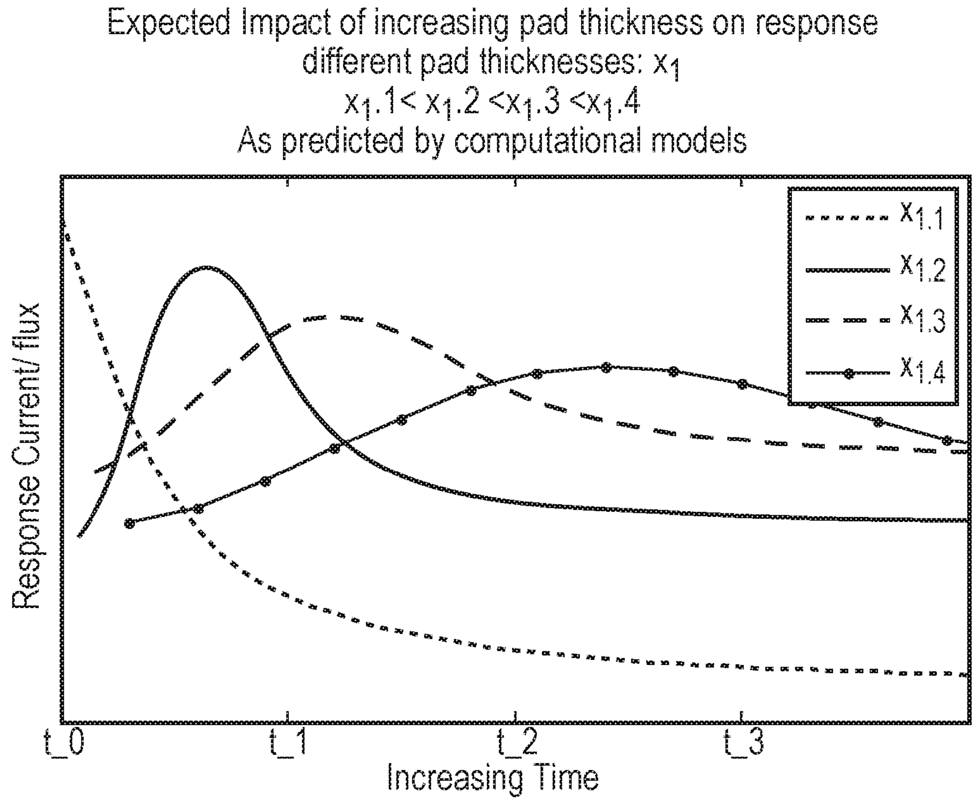
Expected Impact of increasing pad thickness on response
different pad thicknesses: x1
x1.1< x1.2 <x1.3 <x1.4
As predicted by computational models
Response Current/ flux
x1.1
x1.2
x1.3
x1.4
t_0
t_1
t_2
t_3
Increasing Time

FIG. 5b

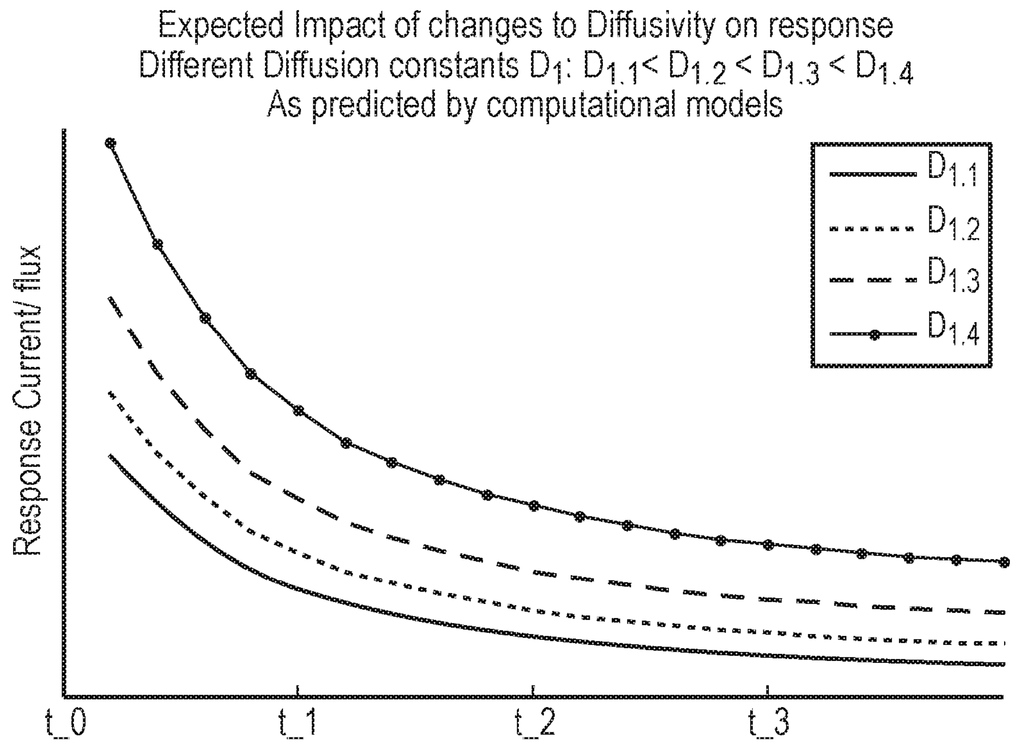
Expected Impact of changes to Diffusivity on response
Different Diffusion constants D1: D1.1< D1.2 < D1.3 < D1.4
As predicted by computational models
Response Current/ flux
D1.1
D1.2
D1.3
D1.4
t_0
t_1
t_2
t_3

FIG. 5c

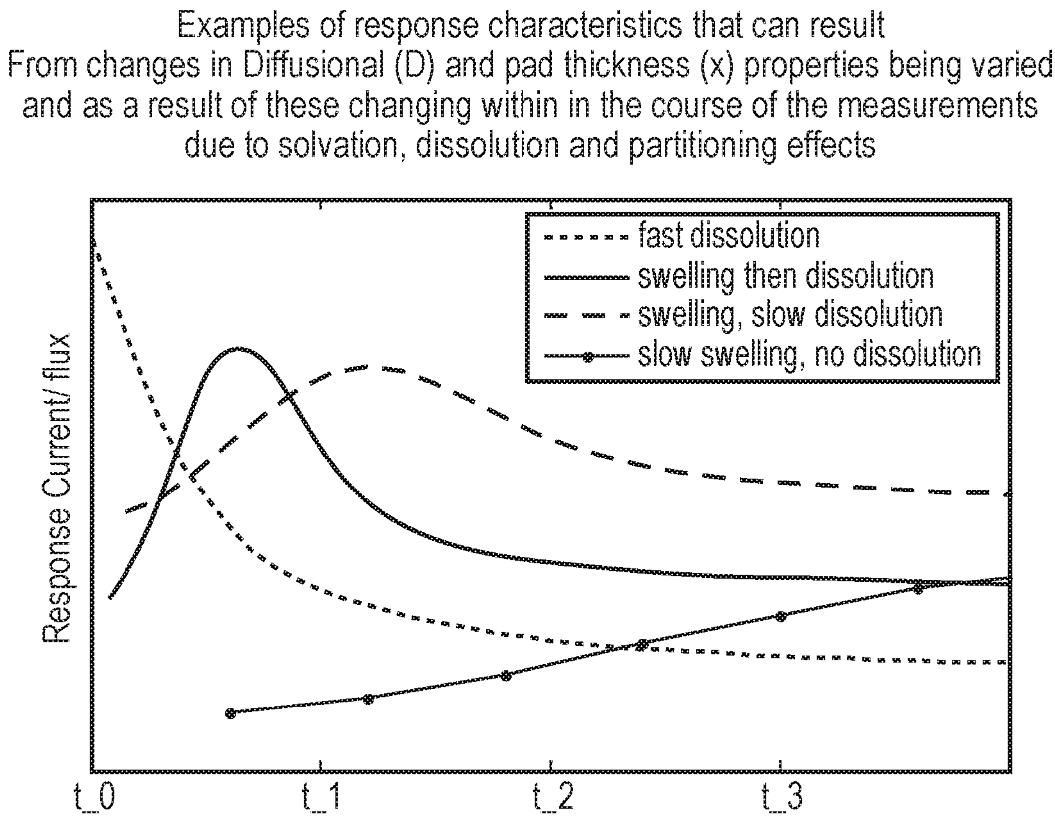
Examples of response characteristics that can result
From changes in Diffusional (D) and pad thickness (x) properties being varied
and as a result of these changing within in the course of the measurements
due to solvation, dissolution and partitioning effects
Response Current/ flux
fast dissolution
swelling then dissolution
swelling, slow dissolution
slow swelling, no dissolution
t_0
t_1
t_2
t_3

FIG. 5d

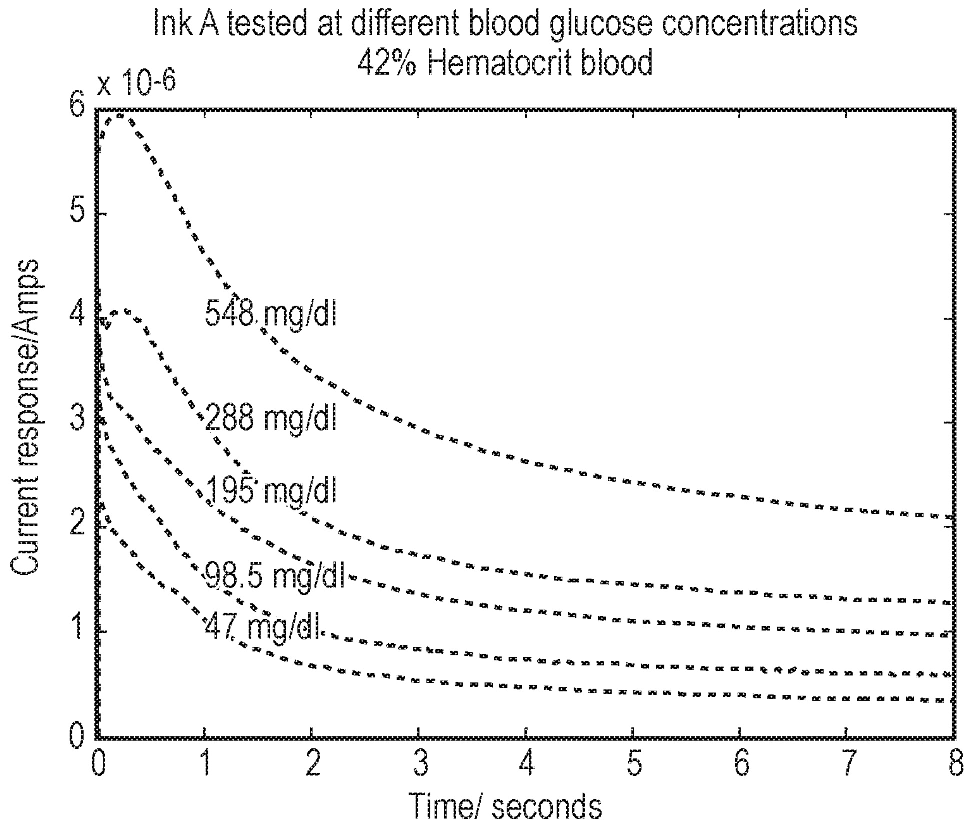
Ink A tested at different blood glucose concentrations
42% Hematocrit blood
x 10-6
Current response/Amps
Time/ seconds
548 mg/dl
288 mg/dl
195 mg/dl
98.5 mg/dl
47 mg/dl
0
1
2
3
4
5
6
7
8

FIG. 6a

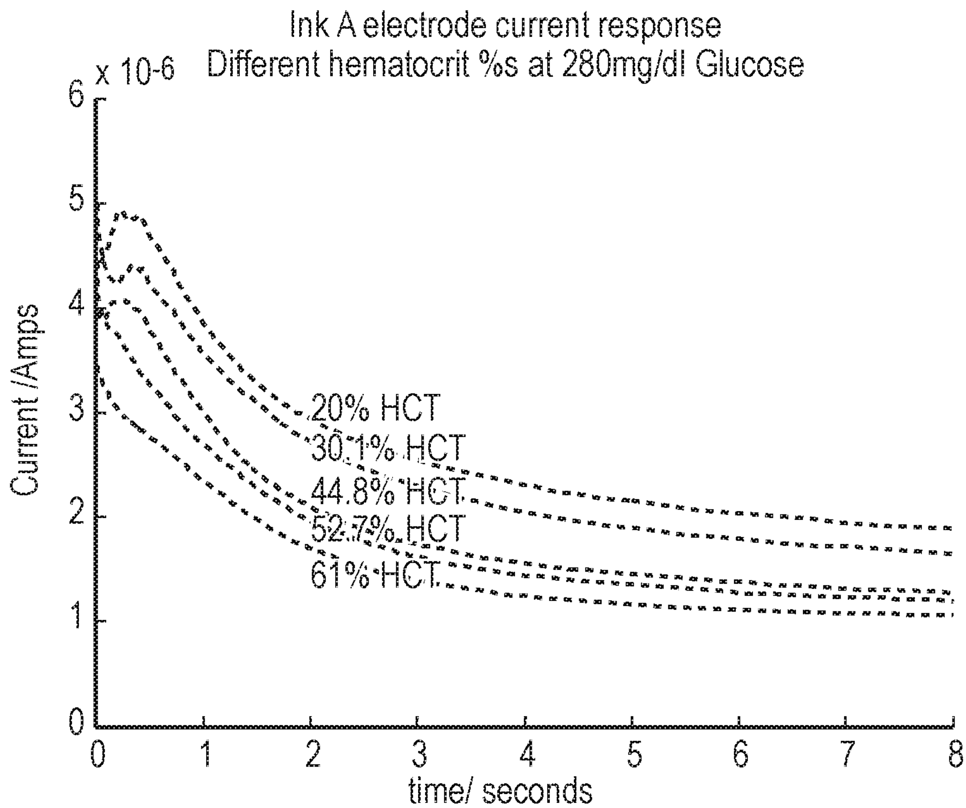
Ink A electrode current response
Different hematocrit %s at 280mg/dl Glucose
x 10-6
Current /Amps
time/ seconds
20% HCT
30.1% HCT
44.8% HCT
52.7% HCT
61% HCT
0
1
2
3
4
5
6
7
8

FIG. 6b

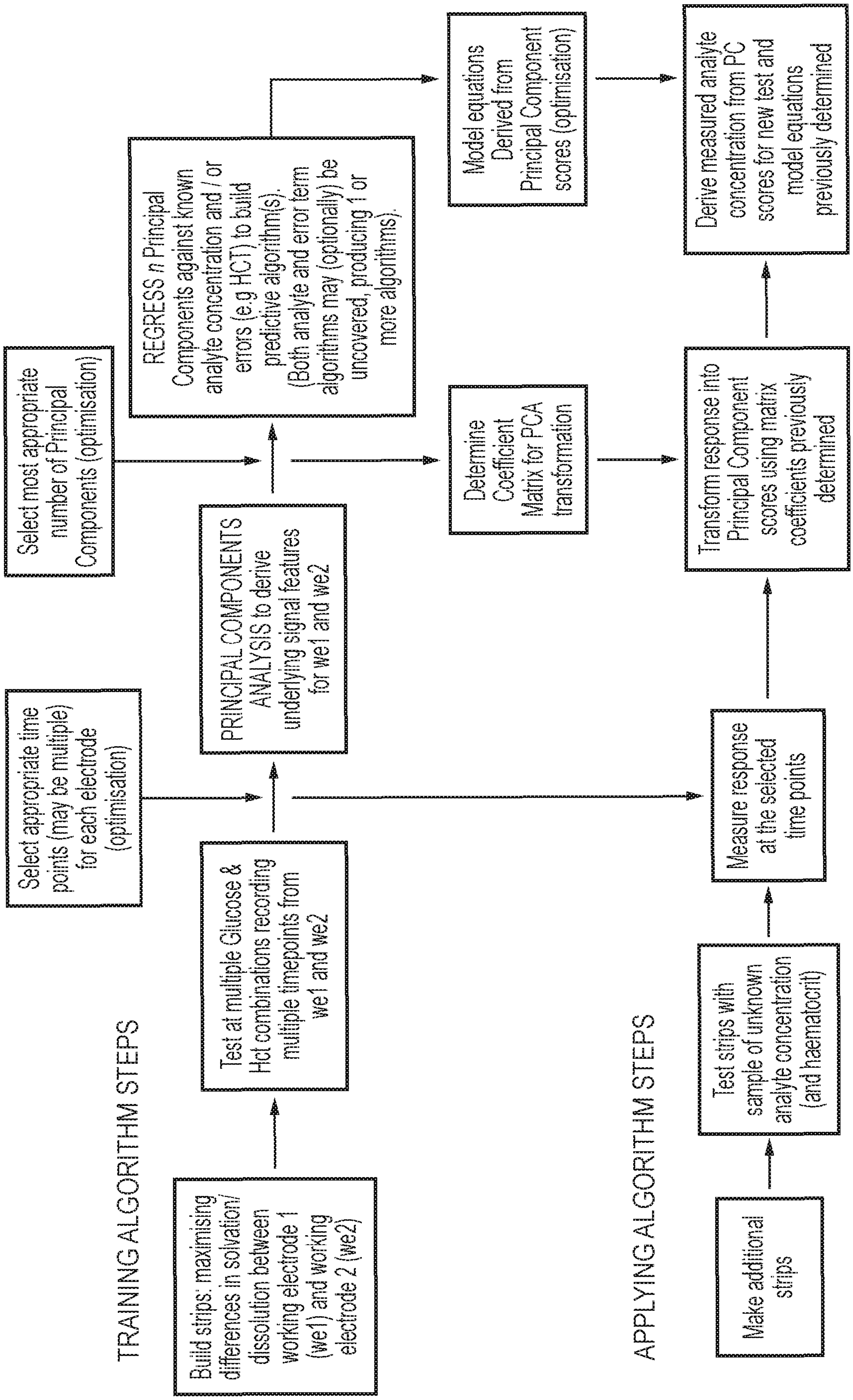
TRAINING ALGORITHM STEPS
Build strips: maximising differences in solvation/ dissolution between working electrode 1 (we1) and working electrode 2 (we2)
Test at multiple Glucose & Hct combinations recording multiple timepoints from we1 and we2
Select appropriate time points (may be multiple) for each electrode (optimisation)
PRINCIPAL COMPONENTS ANALYSIS to derive underlying signal features for we1 and we2
Select most appropriate number of Principal Components (optimisation)
REGRESS n Principal Components against known analyte concentration and / or errors (e.g HCT) to build predictive algorithm(s). (Both analyte and error term algorithms may (optionally) be uncovered, producing 1 or more algorithms).
Determine Coefficient Matrix for PCA transformation
Model equations Derived from Principal Component scores (optimisation)
APPLYING ALGORITHM STEPS
Make additional strips
Test strips with sample of unknown analyte concentration (and haematocrit)
Measure response at the selected time points
Transform response into Principal Component scores using matrix coefficients previously determined
Derive measured analyte concentration from PC scores for new test and model equations previously determined

FIG. 7

Z score conversion coefficients

| | input1 | input2 | input3 | input4 | input5 | input6 | input7 | input8 | input9 | input10 | input11 | input12 | input13 | input14 | input15 | input16 |
|---|---|---|---|---|---|---|---|---|---|---|---|---|---|---|---|---|
| mean | 1.31E-06 | 7.41E-07 | 5.30E-07 | 4.49E-07 | 4.13E-07 | 3.93E-07 | 3.82E-07 | 3.74E-07 | 2.36E-06 | 1.74E-06 | 1.47E-06 | 1.33E-06 | 1.24E-06 | 1.17E-06 | 1.12E-06 | 1.08E-06 |
| std | 2.41E-07 | 2.59E-07 | 2.35E-07 | 2.21E-07 | 2.14E-07 | 2.10E-07 | 2.07E-07 | 2.05E-07 | 1.25E-06 | 1.00E-06 | 8.68E-07 | 7.88E-07 | 7.35E-07 | 6.98E-07 | 6.70E-07 | 6.48E-07 |

Coefficient Matrix

| | input1 | input2 | input3 | input4 | input5 | input6 | input7 | input8 | input9 | input10 | input11 | input12 | input13 | input14 | input15 | input16 |
|---|---|---|---|---|---|---|---|---|---|---|---|---|---|---|---|---|
| input1 | 0.240382 | 0.30105 | 0.752892 | -0.51809 | 0.022654 | -0.07671 | 0.089433 | 0.036413 | -0.00212 | -0.01554 | -0.01335 | 0.008377 | 0.009718 | -0.00176 | -0.00147 | 0.000721 |
| input2 | 0.239045 | 0.55745 | 0.160612 | 0.53187 | -0.04295 | 0.351749 | -0.35241 | -0.21961 | 0.019127 | 0.1511 | 0.03851 | -0.00232 | -0.02633 | -0.00486 | 0.003295 | -0.00428 |
| input3 | 0.247416 | 0.374883 | -0.13882 | 0.273442 | 0.003346 | -0.30028 | 0.27176 | 0.515645 | 0.087223 | -0.51456 | 0.005466 | -0.03098 | 0.022189 | 0.020505 | -0.00084 | -0.00056 |
| input4 | 0.250946 | 0.226386 | -0.23384 | -0.00365 | -0.00777 | -0.34082 | 0.319357 | -0.0797 | -0.44153 | 0.566026 | -0.2522 | -0.16113 | -0.01232 | 0.000938 | 0.001419 | 0.03828 |
| input5 | 0.251861 | 0.139498 | -0.26468 | -0.16864 | 0.00034 | -0.21892 | 0.106466 | -0.3416 | 0.225489 | 0.05163 | 0.566839 | 0.515431 | 0.012165 | -0.00865 | -0.01338 | -0.02964 |
| input6 | 0.252075 | 0.087924 | -0.2727 | -0.25236 | -0.02036 | 0.008352 | -0.05557 | -0.25471 | 0.648575 | -0.04094 | -0.44416 | -0.31602 | 0.027917 | -0.03799 | 0.011807 | -0.01975 |
| input7 | 0.252118 | 0.056231 | -0.26754 | -0.30627 | -0.03048 | 0.194578 | -0.23409 | -0.25804 | -0.56516 | -0.52197 | -0.11984 | 0.024444 | 0.011162 | 0.012592 | 0.023376 | 0.008172 |
| input8 | 0.252071 | 0.030168 | -0.27074 | -0.33228 | -0.04517 | 0.391904 | -0.22839 | 0.619293 | 0.026822 | 0.319034 | 0.225636 | -0.05011 | -0.05913 | 0.024395 | -0.02135 | 0.00733 |
| input9 | 0.252199 | -0.16629 | 0.028706 | -0.09659 | 0.731676 | 0.357568 | 0.404073 | -0.10526 | -0.00236 | -0.04378 | 0.136521 | -0.17411 | -0.09554 | 0.02812 | 0.010095 | 0.011555 |
| input10 | 0.25195 | -0.2026 | 0.056217 | 0.101027 | 0.369149 | -0.1646 | -0.31407 | 0.163012 | -0.00345 | 0.085886 | -0.33035 | 0.430209 | 0.442556 | -0.28683 | -0.10456 | -0.05358 |
| input11 | 0.251567 | -0.22818 | 0.080813 | 0.088982 | 0.115049 | -0.28655 | -0.31709 | 0.016975 | 0.029709 | 0.018645 | -0.05111 | 0.030953 | -0.27725 | 0.762036 | -0.00211 | 0.107939 |
| input12 | 0.251509 | -0.23181 | 0.084572 | 0.090035 | -0.03383 | -0.23196 | -0.20606 | 0.007235 | -0.0109 | -0.01528 | 0.144448 | -0.16727 | -0.36534 | -0.47227 | 0.589774 | 0.129376 |
| input13 | 0.25154 | -0.22939 | 0.085166 | 0.094782 | -0.15092 | -0.13289 | -0.07418 | -0.04533 | -0.04987 | -0.02722 | 0.179403 | -0.27888 | -0.13234 | -0.16224 | -0.50726 | -0.63682 |
| input14 | 0.25156 | -0.22711 | 0.082725 | 0.097476 | -0.24619 | 0.007701 | 0.048848 | -0.06258 | 0.007536 | -0.03756 | 0.168813 | -0.19716 | 0.229449 | -0.10711 | -0.4311 | 0.705834 |
| input15 | 0.251608 | -0.22263 | 0.080279 | 0.103348 | -0.30334 | 0.134374 | 0.179611 | -0.03183 | -0.01045 | 0.016751 | 0.104324 | -0.11269 | 0.599001 | 0.267436 | 0.458946 | -0.24846 |
| input16 | 0.251633 | -0.21749 | 0.076772 | 0.11332 | -0.3644 | 0.313013 | 0.35427 | 0.039339 | 0.042171 | -3.79E-06 | -0.35782 | 0.480289 | -0.38695 | -0.03407 | -0.03441 | -0.01609 |

FIG. 8

| input# | 1 | 2 | 3 | 4 | 5 | 6 | 7 | 8 | 9 | 10 | 11 | 12 | 13 | 14 | 15 | 16 | 17 | 18 | 19 | 20 | 21 |
|---|---|---|---|---|---|---|---|---|---|---|---|---|---|---|---|---|---|---|---|---|---|
| 1 | -0.15996 | 0.746626 | -0.09352 | 0.530578 | -0.25865 | -0.22549 | -0.0112 | -0.01054 | -0.04177 | -0.0714 | 0.005311 | -0.01274 | -0.0283 | -0.01432 | 0.022263 | 0.010962 | -0.00305 | 0.005964 | -0.00415 | 0.001085 | -0.00019 |
| 2 | -0.20795 | 0.390717 | -0.19701 | -0.17497 | 0.28528 | 0.548298 | 0.193353 | 0.051262 | 0.176727 | 0.450407 | -0.03758 | 0.075449 | 0.188835 | 0.004757 | -0.18614 | -0.01776 | 0.016857 | 0.018813 | 0.010627 | -0.00614 | -0.00126 |
| 3 | -0.2215 | 0.187693 | -0.02791 | -0.17694 | 0.343034 | 0.226967 | 0.064846 | -0.03108 | -0.08507 | -0.39416 | 0.188076 | -0.10785 | -0.38121 | 0.061421 | 0.582973 | -0.05444 | -0.0125 | -0.08218 | -0.01833 | 0.02219 | 0.001507 |
| 4 | -0.22478 | 0.080766 | 0.097641 | -0.08164 | 0.302973 | -0.01791 | -0.04715 | -0.01732 | -0.16807 | -0.43242 | -0.52492 | -0.08507 | -0.14128 | 0.023789 | -0.53746 | 0.134094 | -0.05405 | 0.048034 | -0.00984 | -0.03681 | 0.007023 |
| 5 | -0.22466 | 0.027077 | 0.167593 | -0.02373 | 0.266982 | -0.13726 | -0.09462 | -0.02544 | -0.0611 | -0.2082 | 0.697241 | 0.018818 | 0.33901 | -0.27305 | -0.29815 | 0.036754 | 0.073752 | 0.035122 | 0.014976 | 0.004824 | -0.0138 |
| 6 | -0.22413 | -0.00015 | 0.200792 | 0.0172 | 0.239413 | -0.21605 | -0.12863 | 0.014043 | 0.047988 | 0.02355 | -0.37489 | -0.08994 | 0.656695 | 0.035651 | 0.442766 | -0.05523 | 0.008399 | 0.052508 | 0.029968 | 0.039751 | 0.001247 |
| 7 | -0.22364 | -0.01981 | 0.224373 | 0.035105 | 0.209736 | -0.23746 | -0.15622 | 0.059784 | 0.085034 | 0.23823 | -0.07193 | 0.75235 | -0.31855 | -0.13654 | 0.060325 | -0.06886 | -0.05958 | -0.04619 | -0.00894 | -0.01261 | 0.007392 |
| 8 | -0.22321 | -0.03071 | 0.238404 | 0.05335 | 0.19072 | -0.25936 | -0.18867 | 0.046712 | 0.179835 | 0.437169 | 0.127699 | -0.53127 | -0.31917 | 0.333812 | -0.09978 | 0.000304 | 0.036416 | -0.03287 | -0.00417 | -0.02105 | -8.53E-05 |
| 9 | 0.221028 | -0.01474 | 0.216624 | 0.202615 | 0.309479 | -0.18891 | 0.738281 | -0.04467 | -0.37263 | 0.169959 | -0.00774 | -0.03442 | -0.02653 | -0.03535 | 0.004762 | -0.10368 | 0.014551 | 0.036653 | -0.02789 | 0.019006 | 0.005324 |
| 10 | 0.219555 | 0.081167 | 0.350428 | 0.098154 | 0.056473 | 0.095812 | 0.152026 | -0.4215 | 0.474331 | -0.15664 | 0.047823 | 0.148593 | 0.04268 | 0.282359 | 0.015602 | 0.444276 | 0.013183 | -0.01931 | 0.189019 | -0.07805 | -0.06481 |
| 11 | 0.217548 | 0.151145 | 0.37459 | -0.04373 | -0.02999 | 0.117671 | -0.0598 | -0.15751 | 0.293346 | -0.03214 | -0.0666 | -0.19357 | -0.05122 | -0.42021 | -0.03903 | -0.38244 | -0.40924 | -0.05603 | -0.25982 | 0.111804 | 0.194614 |
| 12 | 0.214526 | 0.209175 | 0.38208 | -0.1673 | -0.08866 | 0.104521 | -0.11707 | 0.147034 | -0.04181 | 0.005774 | -0.10428 | -0.03933 | -0.07693 | -0.19793 | 0.018699 | -0.09119 | 0.751673 | 0.005899 | -0.01402 | -0.03714 | -0.21807 |
| 13 | 0.211021 | 0.256984 | 0.380622 | -0.26802 | -0.12922 | 0.074699 | -0.11121 | 0.453027 | -0.32819 | 0.020195 | 0.115805 | 0.097768 | 0.094351 | 0.338916 | 0.003852 | 0.141876 | -0.37239 | 0.034295 | 0.104824 | -0.01061 | 0.083039 |
| 14 | -0.21932 | -0.17402 | 0.213515 | 0.28492 | -0.12618 | 0.30068 | 0.010414 | 0.0226 | -0.16802 | 0.003826 | -0.01247 | -0.00013 | 0.098647 | 0.0314 | -0.01669 | 0.006219 | 0.017348 | -0.68982 | -0.14869 | -0.38213 | 0.064644 |
| 15 | -0.21936 | -0.17431 | 0.214681 | 0.278412 | -0.1269 | 0.312458 | -0.01526 | 0.00576 | -0.12036 | 0.020873 | -0.01295 | -0.00844 | -0.03871 | -0.01765 | -0.02098 | 0.048528 | -0.00775 | -0.00925 | 0.21451 | 0.773438 | -0.14058 |
| 16 | -0.21945 | -0.1752 | 0.214801 | 0.268567 | -0.12914 | 0.320612 | -0.02238 | -0.00596 | -0.09238 | 0.031656 | 0.018831 | -0.02612 | -0.06877 | -0.07948 | 0.092101 | 0.044507 | -0.03528 | 0.699289 | -0.09183 | -0.38385 | 0.069067 |
| 17 | 0.220977 | -0.09952 | -0.09907 | 0.35878 | 0.284467 | 0.052298 | 0.022274 | 0.67794 | 0.347601 | -0.12357 | -0.02399 | -0.08402 | -0.04037 | -0.22882 | 0.041566 | 0.23167 | -0.03586 | -0.03517 | 0.052055 | -0.02225 | -0.01114 |
| 18 | 0.224168 | -0.02916 | -0.05627 | 0.273357 | 0.228252 | 0.127477 | -0.17867 | 0.017749 | 0.031833 | -0.15356 | 0.079395 | 0.187832 | 0.098266 | 0.519191 | -0.10534 | -0.35721 | 0.171429 | 0.097667 | -0.45959 | 0.155564 | 0.109465 |
| 19 | 0.22526 | 0.007191 | -0.05807 | 0.196051 | 0.210578 | 0.114075 | -0.2565 | -0.132 | -0.12018 | 0.010093 | 0.001933 | -0.01872 | -0.00223 | 0.020764 | -0.04199 | -0.40091 | -0.18702 | -0.01325 | 0.525025 | -0.23298 | -0.4732 |
| 20 | 0.225703 | 0.034485 | -0.069 | 0.126081 | 0.204045 | 0.084874 | -0.28731 | -0.18646 | -0.22953 | 0.125952 | -0.02487 | -0.02868 | -0.03742 | -0.12039 | 0.040039 | 0.12899 | 0.169913 | -0.02999 | 0.331306 | 0.029079 | 0.718717 |
| 21 | 0.225726 | 0.057151 | -0.08192 | 0.062774 | 0.202887 | 0.047568 | -0.3019 | -0.21304 | -0.29387 | 0.216218 | -0.01504 | -0.04953 | -0.01092 | -0.18298 | 0.10026 | 0.47289 | -0.13628 | -0.02194 | -0.4551 | 0.063855 | -0.34908 |

FIG. 9

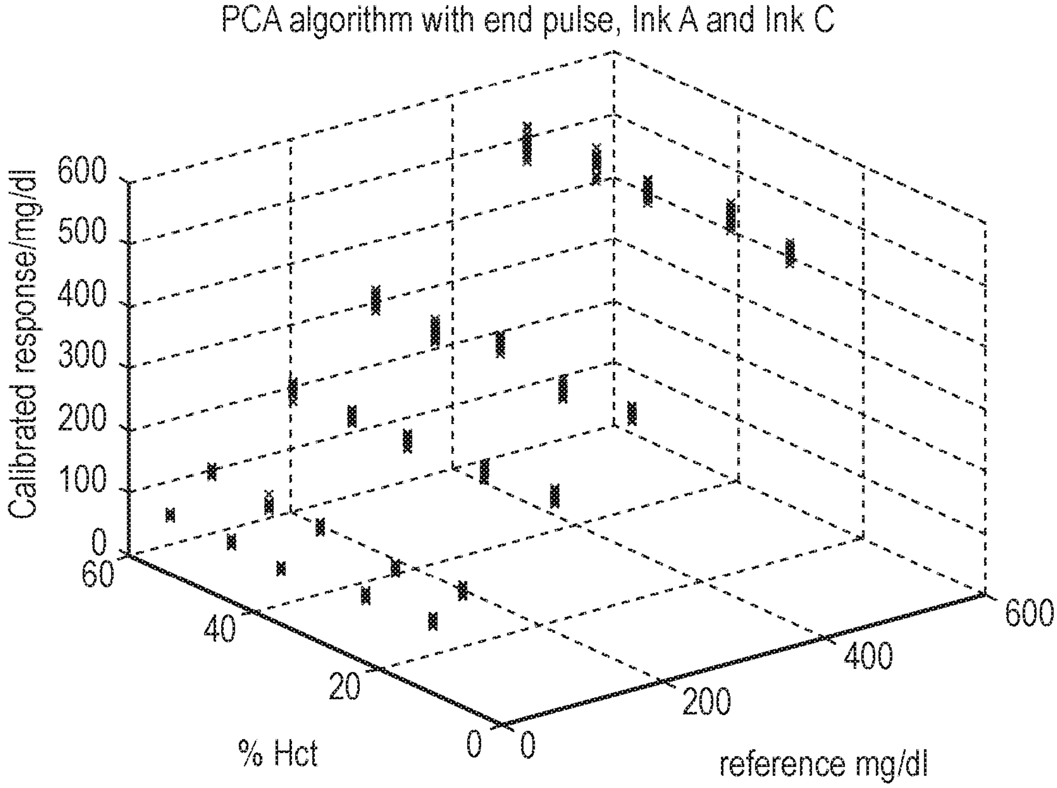


FIG. 10a

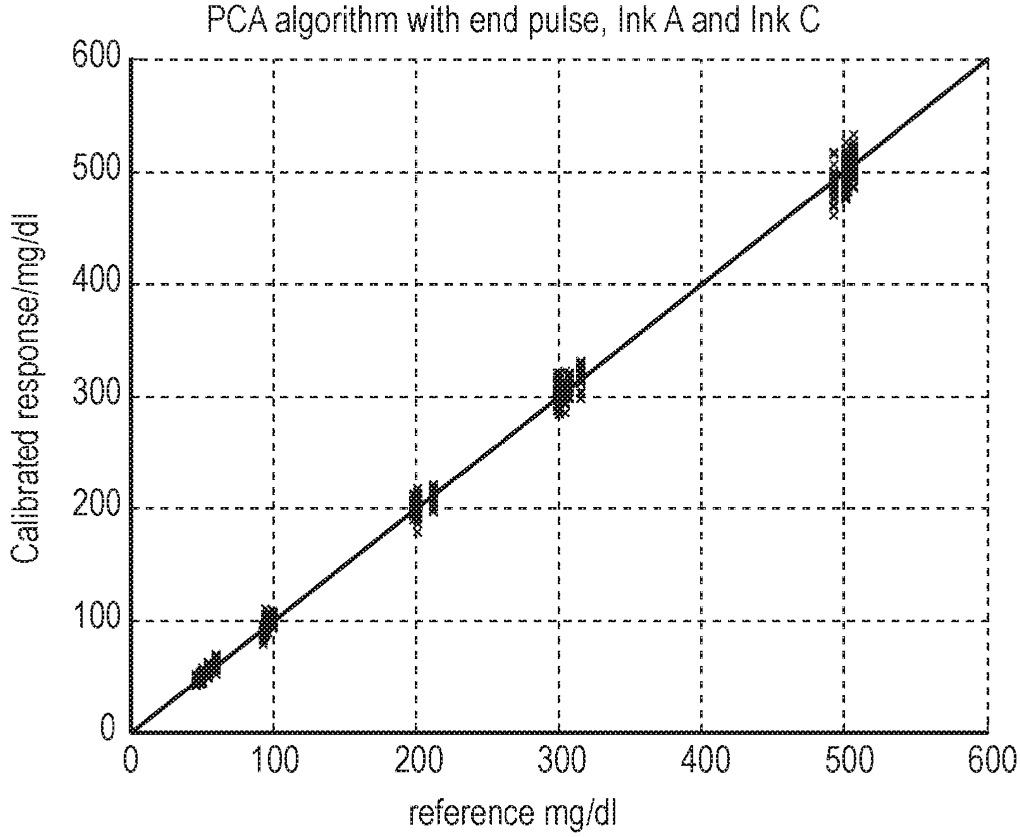


FIG. 10b

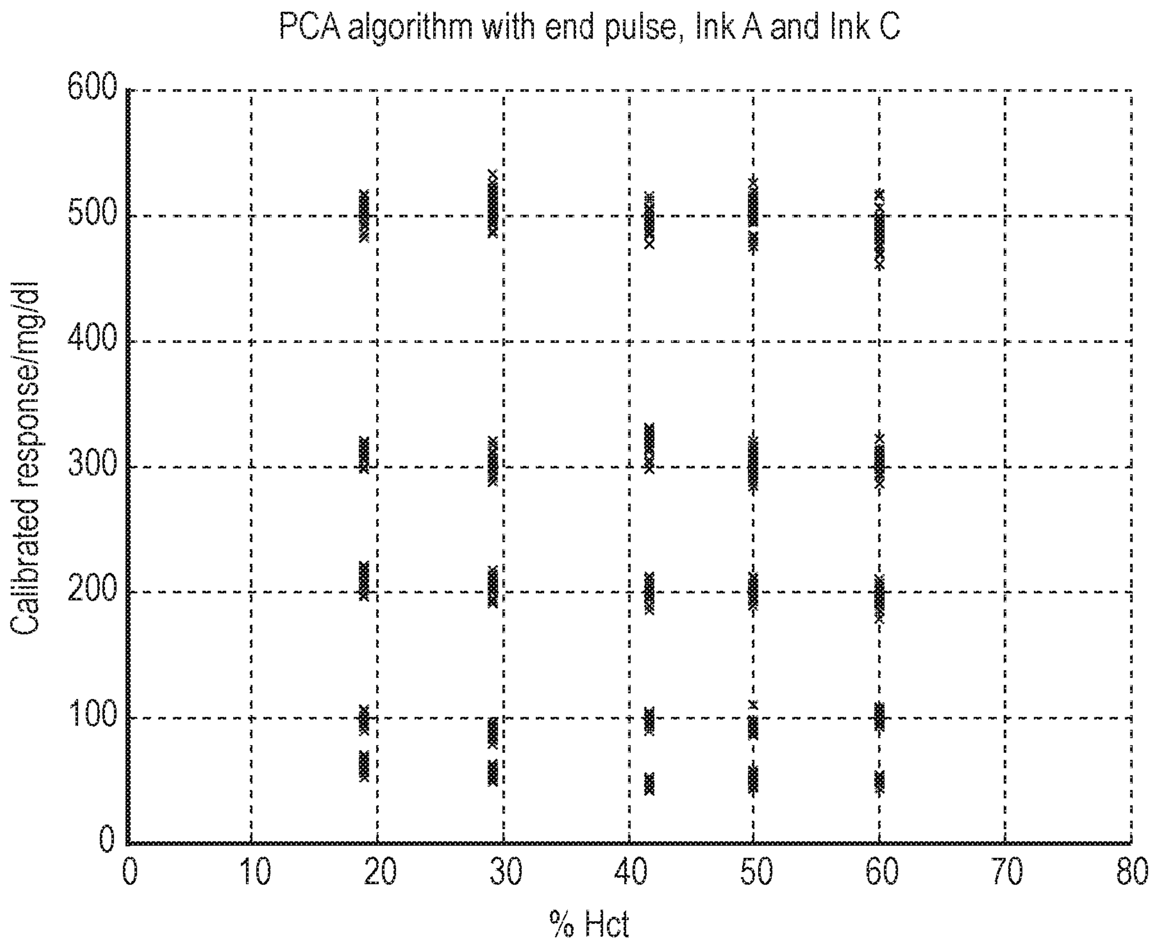
PCA algorithm with end pulse, Ink A and Ink C
Calibrated response/mg/dl
0
100
200
300
400
500
600
0
10
20
30
40
50
60
70
80
% Hct

FIG. 10c

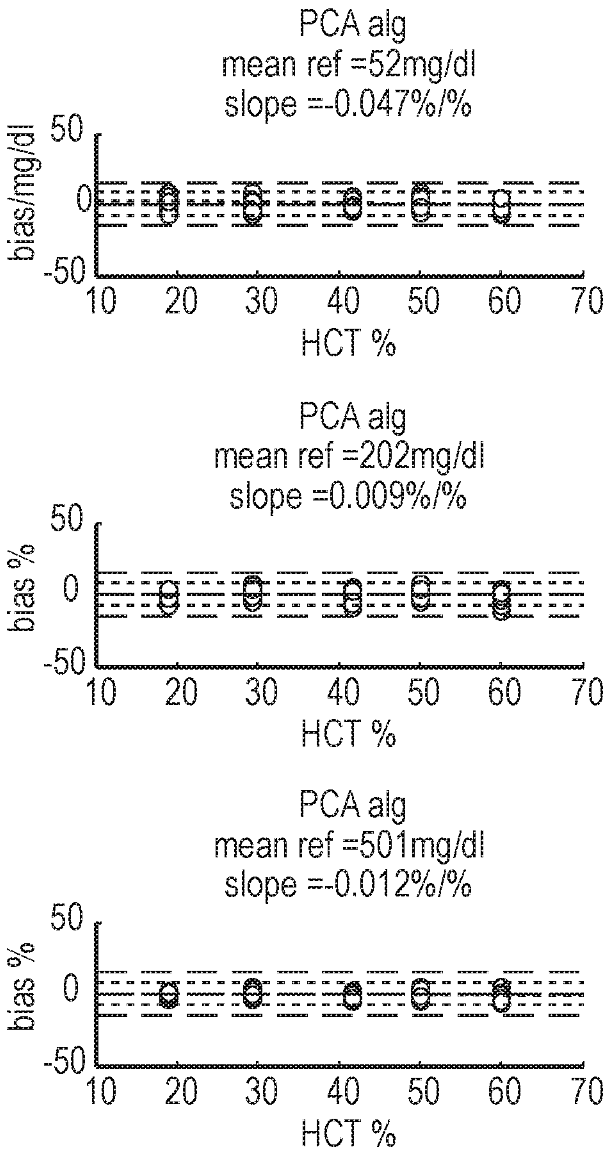


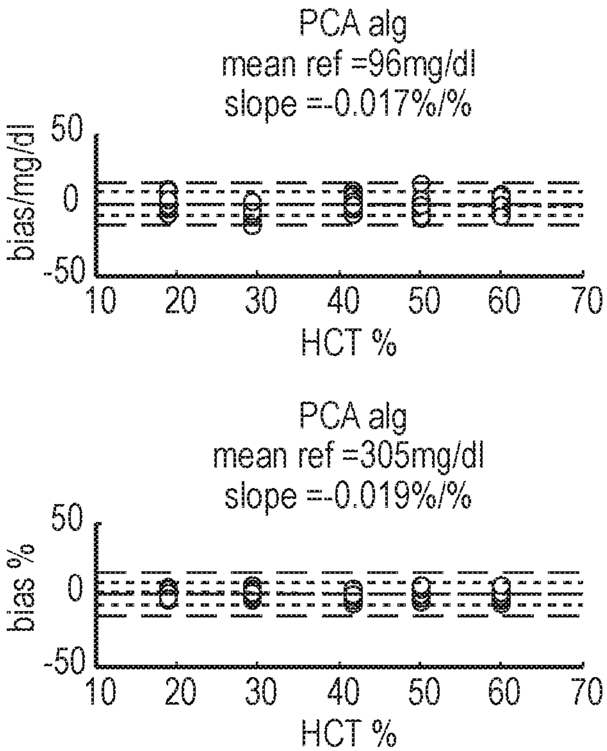


N=660
Mean bias 0.1%

| | | | | | |
|---|---|---|---|---|---|
| Hct mean | 19.00 | 29.20 | 41.60 | 50.00 | 60.00 |
| No. @Hct | 127 | 134 | 129 | 136 | 134 |
| % <15% | 100.00 | 99.25 | 100.00 | 98.53 | 100.00 |

| | | | | | |
|---|---|---|---|---|---|
| glu mean | 52 | 96 | 202 | 305 | 501 |
| No. @glu | 133 | 130 | 133 | 128 | 136 |
| % <15% | 99.25 | 98.46 | 100.00 | 100.00 | 100.00 |

FIG. 10d

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2056107 A
- US 20070131549 A
- US 2004180391 A1
- EP 0537761 A2
- EP 2444500 A2